# EUROPEAN PATENT APPLICATION

(11) **EP 3 804 764 A1**
(43) Date of publication of application: **14.04.2021**
(21) Application number: 19812523.9
(22) Date of filing: 27.05.2019
(51) Int. Cl.: A61K 47/68, A61K 39/395, A61P 35/00

(54) **TREATMENT OF HER2-MUTATED CANCER BY ADMINISTERING ANTI-HER2 ANTIBODY-DRUG CONJUGATE**

(30) Priority: 28.05.2018 JP 2018101211; 21.09.2018 JP 2018177132
(71) Applicant: Daiichi Sankyo Company, Limited, Tokyo 103-8426 (JP)
(72) Inventor: FUJISAKI, Yoshihiko, Tokyo 103-8426 (JP); SATO, Yuta, Tokyo 103-8426 (JP); SERIZAWA, Yui, Tokyo 103-8426 (JP); SHIGA, Ryota, Tokyo 103-8426 (JP); SAITO, Kaku, Tokyo 103-8426 (JP); JIKOH, Takahiro, Tokyo 103-8426 (JP); TOKUHIRO, Shinya, Tokyo 103-8426 (JP)
(74) Representative: Wallace, Sheila Jane
(86) International application number: PCT/JP2019/020886
(87) International publication number: WO 2019/230645

(57) **Abstract**

A therapeutic agent for HER2-mutated cancer comprising, as an active component, an anti-HER2 antibody-drug conjugate in which a drug-linker represented by the following formula (wherein A represents a connecting position to an anti-HER2 antibody) is conjugated to the antibody via a thioether bond and/or a method of treatment for cancer, comprising administering the anti-HER2 antibody-drug conjugate to a subject determined to have HER2-mutated cancer.

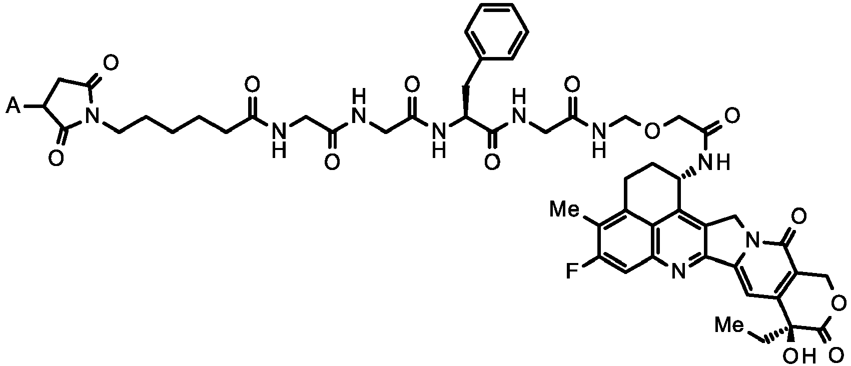

## Description

### Technical Field

The present invention relates to a therapeutic agent for HER2-mutated cancer comprising a specific anti-HER2 antibody-drug conjugate and/or a method of treatment for cancer comprising administering the specific anti-HER2 antibody-drug conjugate to a subject determined to have HER2-mutated cancer.

### Background Art

Human epidermal growth factor receptor 2 (HER2) is a transmembrane receptor belonging to the epidermal growth factor receptor subfamily of receptor protein tyrosine kinases (Non Patent References 1 to 6).

HER2 is overexpressed in various cancer types such as breast cancer and gastric cancer (Non Patent References 7 to 12) and has been reported to be a negative prognostic factor in breast cancer (Non Patent References 13 and 14). As anti-HER2 drugs effective for HER2-overexpressing cancers, trastuzumab, trastuzumab emtansine, pertuzumab, lapatinib, and the like are known.

Meanwhile, HER2 has mutants, which are known to be one of the cancer driver mutations. Such HER2-mutated cancers have been reported to be present, for example, at a percentage of about 2% to 3% in non-small cell lung cancers (Non Patent References 15 to 20). Studies have been conducted to verify the effects of anti-HER2 drugs on HER2-mutated cancers (Non Patent References 21 and 22).

An antibody-drug conjugate (ADC) having a drug with cytotoxicity conjugated to an antibody, whose antigen is expressed on the surface of cancer cells and which also binds to an antigen capable of cellular internalization, and therefore can deliver the drug selectively to cancer cells, is thus expected to cause accumulation of the drug within cancer cells and to kill the cancer cells (Non Patent References 23 to 27).

As one such antibody-drug conjugate, an antibody-drug conjugate comprising an anti-HER2 antibody and a derivative of exatecan, which is a topoisomerase I inhibitor, as its components is known (Patent References 1 to 3 and Non Patent References 28 to 31).

### Citation List

### Patent Literature

Patent Reference 1 International Publication No. WO 2015/115091
Patent Reference 2 International Publication No. WO 2015/155976
Patent Reference 3 International Publication No. WO 2018/066626

### Non Patent Literature

Non Patent Reference 1: Coussens L, et al., Science. 1985; 230(4730): 1132-1139.
Non Patent Reference 2: Graus-Porta G, et al., EMBO J. 1997;16:1647-1655.
Non Patent Reference 3: Karnagaran D, et al., EMBO J. 1996;15:254-264.
Non Patent Reference 4: Sliwkowski MX, et al., J Biom Chem. 1994; 269: 14661-14665.
Non Patent Reference 5: Di Fore PP, et al., Science. 1987; 237: 178-182.
Non Patent Reference 6: Hudziak RM, et al., Proc Natl Acad Sci U S A. 1987; 84: 7159-7163.
Non Patent Reference 7: Hardwick R, et al., Eur. J Surg Oncol. 1997 (23):30-35.
Non Patent Reference 8: Korkaya H, et al., Oncogene. 2008;27 (47) :6120-6130.
Non Patent Reference 9: Yano T, et al., Oncol Rep. 2006; 15(1): 65-71.
Non Patent Reference 10: Slamon DJ, et al., Science. 1987; 235: 177-182.
Non Patent Reference 11: Gravalos C, et al., Ann Oncol 19: 1523-1529, 2008.
Non Patent Reference 12: Fukushige S et al., Mol Cell Biol 6: 955-958, 1986.
Non Patent Reference 13: Slamon DJ, et al., Science. 1989; 244: 707-712.
Non Patent Reference 14: Kaptain S, et al., Diagn Mol Pathol 10: 139-152, 2001.
Non Patent Reference 15: Mazieres J, et al., J Clin Oncol 2013; 31: 1997-2003.
Non Patent Reference 16: Arcila ME, et al., Clin Cancer Res 2012; 18: 4910-8.
Non Patent Reference 17: Li C, et al., J Thorac Oncol 2012; 7: 85-9.
Non Patent Reference 18: Tomizaka K, et al., Lung Cancer 2011; 74: 139-44.
Non Patent Reference 19: Shigematsu H, et al., Cancer Res 2005; 65: 1642-6.
Non Patent Reference 20: Yokoyama T, et al., Cancer Sci 2006; 97: 753-9.
Non Patent Reference 21: Connell CM, et al., ESMO Open 2017; 2:e000279.
Non Patent Reference 22: M.G.Kris, et al., Annals of Oncology 26: 1421-1427, 2015.
Non Patent Reference 23: Ducry, L., et al., Bioconjugate Chem. (2010) 21, 5-13.
Non Patent Reference 24: Alley, S. C., et al., Current Opinion in Chemical Biology (2010) 14, 529-537.
Non Patent Reference 25: Damle N. K. Expert Opin. Biol. Ther. (2004) 4, 1445-1452.
Non Patent Reference 26: Senter P. D., et al., Nature Biotechnology (2012) 30, 631-637.
Non Patent Reference 27: Howard A. et al., J Clin Oncol 29: 398-405.
Non Patent Reference 28: Ogitani Y. et al., Clinical Cancer Research (2016) 22(20), 5097-5108.
Non Patent Reference 29: Ogitani Y. et al., Cancer Science (2016) 107, 1039-1046.
Non Patent Reference 30: Doi T, et al., Lancet Oncol 2017; 18: 1512-22.
Non Patent Reference 31: Takegawa N, et al., Int. J. Cancer: 141, 1682-1689 (2017)

### Summary of Invention

### Technical Problem

The anti-HER2 antibody-drug conjugate comprising an anti-HER2 antibody and a derivative of exatecan, which is a topoisomerase I inhibitor, as its components is known to exert an antitumor effect against cancers determined to have HER2 overexpression. However, it has not been demonstrated that the anti-HER2 antibody-drug conjugate can exert an antitumor effect on HER2-mutated cancers. An object of the present invention is to provide a therapeutic agent for HER2-mutated cancer comprising a specific anti-HER2 antibody-drug conjugate and/or a method of treatment for cancer comprising administering the specific anti-HER2 antibody-drug conjugate to a subject determined to have HER2-mutated cancer.

### Solution to Problem

As a result of diligent studies in order to solve the above problems, the present inventors have found that a specific anti-HER2 antibody-drug conjugate exhibits an excellent antitumor effect on HER2-mutated cancers, thereby accomplishing the present invention.

Thus, the present invention provides the following [1] to [176].
[1] A therapeutic agent for HER2-mutated cancer comprising, as an active component, an anti-HER2 antibody-drug conjugate in which a drug-linker represented by the following formula: 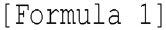 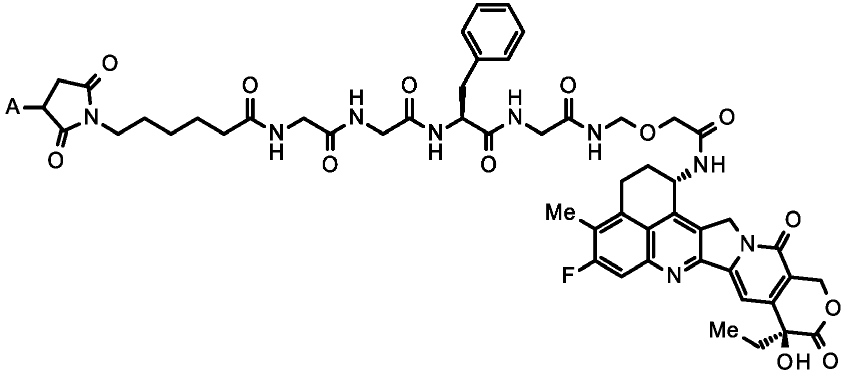 wherein A represents a connecting position to an anti-HER2 antibody;
   is conjugated to the anti-HER2 antibody via a thioether bond.
[2] The therapeutic agent according to [1], wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, I613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770_A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.
[3] The therapeutic agent according to [2], wherein the HER2 mutation in the HER2-mutated cancer is at least one selected from the group consisting of Y772_A775dup, G778_P780dup, G776delinsVC, L755S, V777L, and S310F.
[4] The therapeutic agent according to [1], wherein the HER2 mutation in the HER2-mutated cancer is an exon 20 insertion mutation.
[5] The therapeutic agent according to [4], wherein the exon 20 insertion mutation is at least one selected from the group consisting of Y772_A775dup, G778_P780dup, G776delinsVC, E770 _A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.
[6] The therapeutic agent according to [5], wherein the exon 20 insertion mutation is at least one selected from the group consisting of Y772_A775dup, G778_P780dup, and G776delinsVC.
[7] The therapeutic agent according to [1], wherein the HER2 mutation in the HER2-mutated cancer is a single base pair substitution mutation at a transmembrane domain of a HER2 protein.
[8] The therapeutic agent according to [7], wherein the single base pair substitution mutation at the transmembrane domain of the HER2 protein is at least one selected from the group consisting of V659E, G660D, I654V, I655V, and I661V.
[9] The therapeutic agent according to [8], wherein the single base pair substitution mutation at the transmembrane domain of the HER2 protein is G660D.
[10] The therapeutic agent according to [1], wherein the HER2 mutation in the HER2-mutated cancer is a single base pair substitution mutation at an extracellular domain of a HER2 protein.
[11] The therapeutic agent according to [10], wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.
[12] The therapeutic agent according to [11], wherein the single base pair substitution mutation at the extracellular domain of the HER2 protein is S310F.
[13] The therapeutic agent according to any one of [1] to [12], wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.
[14] The therapeutic agent according to any one of [1] to [12], wherein the cancer is non-small cell lung cancer.
[15] The therapeutic agent according to [14], wherein the non-small cell lung cancer is unresectable and/or metastatic non-small cell lung cancer.
[16] The therapeutic agent according to any one of [1] to [15], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[17] The therapeutic agent according to any one of [1] to [15], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[18] The therapeutic agent according to any one of [1] to [17], wherein the average number of units of the drug-linker conjugated per antibody molecule in the anti-HER2 antibody-drug conjugate is in the range of from 7 to 8.
[19] The therapeutic agent according to any one of [1] to [17], wherein the average number of units of the drug-linker conjugated per antibody molecule in the anti-HER2 antibody-drug conjugate is in the range of from 7.5 to 8.
[20] The therapeutic agent according to any one of [1] to [19], wherein a dose per administration of the anti-HER2 antibody-drug conjugate is in a range of 5.4 mg/kg to 8 mg/kg.
[21] The therapeutic agent according to any one of [1] to [19], wherein a dose per administration of the anti-HER2 antibody-drug conjugate is 6.4 mg/kg.
[22] The therapeutic agent according to any one of [1] to [21], wherein the anti-HER2 antibody-drug conjugate is administered once every 3 weeks.
[23] A therapeutic agent for HER2-mutated cancer comprising, as an active component, an anti-HER2 antibody-drug conjugate represented by the following formula: 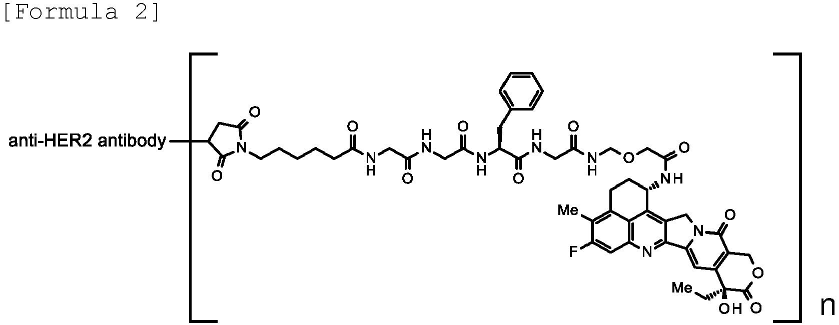 wherein the drug-linker is conjugated to the anti-HER2 antibody via a thioether bond, and n is the average number of units of the drug-linker conjugated per antibody molecule.
[24] The therapeutic agent according to [23], wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, I613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770 _A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.
[25] The therapeutic agent according to [24], wherein the HER2 mutation in the HER2-mutated cancer is at least one selected from the group consisting of Y772_A775dup, G778_P780dup, G776delinsVC, L755S, V777L, and S310F.
[26] The therapeutic agent according to [23], wherein the HER2 mutation in the HER2-mutated cancer is an exon 20 insertion mutation.
[27] The therapeutic agent according to [26], wherein the exon 20 insertion mutation is at least one selected from the group consisting of Y772_A775dup, G778_P780dup, G776delinsVC, E770 _A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.
[28] The therapeutic agent according to [27], wherein the exon 20 insertion mutation is at least one selected from the group consisting of Y772_A775dup, G778_P780dup, and G776delinsVC.
[29] The therapeutic agent according to [23], wherein the HER2 mutation in the HER2-mutated cancer is a single base pair substitution mutation at a transmembrane domain of a HER2 protein.
[30] The therapeutic agent according to [29], wherein the single base pair substitution mutation at the transmembrane domain of the HER2 protein is at least one selected from the group consisting of V659E, G660D, I654V, I655V, and I661V.
[31] The therapeutic agent according to [30], wherein the single base pair substitution mutation at the transmembrane domain of the HER2 protein is G660D.
[32] The therapeutic agent according to [23], wherein the HER2 mutation in the HER2-mutated cancer is a single base pair substitution mutation at an extracellular domain of a HER2 protein.
[33] The therapeutic agent according to [32], wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.
[34] The therapeutic agent according to [33], wherein the single base pair substitution mutation at the extracellular domain of the HER2 protein is S310F.
[35] The therapeutic agent according to any one of [23] to [34], wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.
[36] The therapeutic agent according to any one of [23] to [34], wherein the cancer is non-small cell lung cancer.
[37] The therapeutic agent according to [36], wherein the non-small cell lung cancer is unresectable and/or metastatic non-small cell lung cancer.
[38] The therapeutic agent according to any one of [23] to [37], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[39] The therapeutic agent according to any one of [23] to [37], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[40] The therapeutic agent according to any one of [23] to [39], wherein the average number of units of the drug-linker conjugated per antibody molecule in the anti-HER2 antibody-drug conjugate is in the range of from 7 to 8.
[41] The therapeutic agent according to any one of [23] to [39], wherein the average number of units of the drug-linker conjugated per antibody molecule in the anti-HER2 antibody-drug conjugate is in the range of from 7.5 to 8.
[42] The therapeutic agent according to any one of [23] to [41], wherein a dose per administration of the anti-HER2 antibody-drug conjugate is in a range of 5.4 mg/kg to 8 mg/kg.
[43] The therapeutic agent according to any one of [23] to [41], wherein a dose per administration of the anti-HER2 antibody-drug conjugate is 6.4 mg/kg.
[44] The therapeutic agent according to any one of [23] to [43], wherein the anti-HER2 antibody-drug conjugate is administered once every 3 weeks.
[45] A method of treatment for cancer, comprising administering an anti-HER2 antibody-drug conjugate in which a drug-linker represented by the following formula: 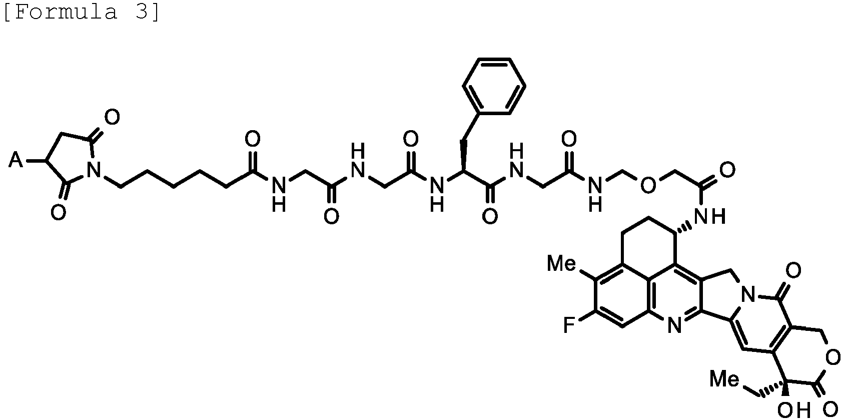 wherein A represents a connecting position to an anti-HER2 antibody;
   is conjugated to the anti-HER2 antibody via a thioether bond, to a subject determined to have HER2-mutated cancer.
[46] The method of treatment according to [45], wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, I613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770_A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.
[47] The method of treatment according to [46], wherein the HER2 mutation in the HER2-mutated cancer is at least one selected from the group consisting of Y772_A775dup, G778_P780dup, G776delinsVC, L755S, V777L, and S310F.
[48] The method of treatment according to [45], wherein the HER2 mutation in the HER2-mutated cancer is an exon 20 insertion mutation.
[49] The method of treatment according to [48], wherein the exon 20 insertion mutation is at least one selected from the group consisting of Y772_A775dup, G778_P780dup, G776delinsVC, E770_A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.
[50] The method of treatment according to [49], wherein the exon 20 insertion mutation is at least one selected from the group consisting of Y772_A775dup, G778_P780dup, and G776delinsVC.
[51] The method of treatment according to [45], wherein the HER2 mutation in the HER2-mutated cancer is a single base pair substitution mutation at a transmembrane domain of a HER2 protein.
[52] The method of treatment according to [51], wherein the single base pair substitution mutation at the transmembrane domain of the HER2 protein is at least one selected from the group consisting of V659E, G660D, I654V, I655V, and I661V.
[53] The method of treatment according to [52], wherein the single base pair substitution mutation at the transmembrane domain of the HER2 protein is G660D.
[54] The method of treatment according to [45], wherein the HER2 mutation in the HER2-mutated cancer is a single base pair substitution mutation at an extracellular domain of a HER2 protein.
[55] The method of treatment according to [54], wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.
[56] The method of treatment according to [55], wherein the single base pair substitution mutation at the extracellular domain of the HER2 protein is S310F.
[57] The method of treatment according to any one of [45] to [56], wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.
[58] The method of treatment according to any one of [45] to [56], wherein the cancer is non-small cell lung cancer.
[59] The method of treatment according to [58], wherein the non-small cell lung cancer is unresectable and/or metastatic non-small cell lung cancer.
[60] The method of treatment according to any one of [45] to [59], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[61] The method of treatment according to any one of [45] to [59], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[62] The method of treatment according to any one of [45] to [61], wherein the average number of units of the drug-linker conjugated per antibody molecule in the anti-HER2 antibody-drug conjugate is in the range of from 7 to 8.
[63] The method of treatment according to any one of [45] to [61], wherein the average number of units of the drug-linker conjugated per antibody molecule in the anti-HER2 antibody-drug conjugate is in the range of from 7.5 to 8.
[64] The method of treatment according to any one of [45] to [63], wherein a dose per administration of the anti-HER2 antibody-drug conjugate is in a range of 5.4 mg/kg to 8 mg/kg.
[65] The method of treatment according to any one of [45] to [63], wherein a dose per administration of the anti-HER2 antibody-drug conjugate is 6.4 mg/kg.
[66] The method of treatment according to any one of [45] to [65], wherein the anti-HER2 antibody-drug conjugate is administered once every 3 weeks.
[67] A method of treatment for cancer, comprising administering an anti-HER2 antibody-drug conjugate represented by the following formula: 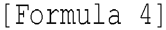 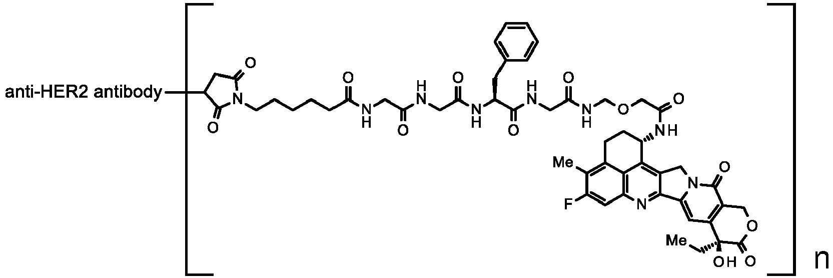 wherein the drug-linker is conjugated to the anti-HER2 antibody via a thioether bond, and n is the average number of units of the drug-linker conjugated per antibody molecule;
   to a subject determined to have HER2-mutated cancer.
[68] The method of treatment according to [67], wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, I613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770 _A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.
[69] The method of treatment according to [68], wherein the HER2 mutation in the HER2-mutated cancer is at least one selected from the group consisting of Y772_A775dup, G778_P780dup, G776delinsVC, L755S, V777L, and S310F.
[70] The method of treatment according to [67], wherein the HER2 mutation in the HER2-mutated cancer is an exon 20 insertion mutation.
[71] The method of treatment according to [70], wherein the exon 20 insertion mutation is at least one selected from the group consisting of Y772_A775dup, G778_P780dup, G776delinsVC, E770 _A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.
[72] The method of treatment according to [71], wherein the exon 20 insertion mutation is at least one selected from the group consisting of Y772_A775dup, G778_P780dup, and G776delinsVC.
[73] The method of treatment according to [67], wherein the HER2 mutation in the HER2-mutated cancer is a single base pair substitution mutation at a transmembrane domain of a HER2 protein.
[74] The method of treatment according to [73], wherein the single base pair substitution mutation at the transmembrane domain of the HER2 protein is at least one selected from the group consisting of V659E, G660D, I654V, I655V, and I661V.
[75] The method of treatment according to [74], wherein the single base pair substitution mutation at the transmembrane domain of the HER2 protein is G660D.
[76] The method of treatment according to [67], wherein the HER2 mutation in the HER2-mutated cancer is a single base pair substitution mutation at an extracellular domain of a HER2 protein.
[77] The method of treatment according to [76], wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.
[78] The method of treatment according to [77], wherein the single base pair substitution mutation at the extracellular domain of the HER2 protein is S310F.
[79] The method of treatment according to any one of [67] to [78], wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.
[80] The method of treatment according to any one of [67] to [78], wherein the cancer is non-small cell lung cancer.
[81] The method of treatment according to [80], wherein the non-small cell lung cancer is unresectable and/or metastatic non-small cell lung cancer.
[82] The method of treatment according to any one of [67] to [81], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[83] The method of treatment according to any one of [67] to [81], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[84] The method of treatment according to any one of [67] to [83], wherein the average number of units of the drug-linker conjugated per antibody molecule in the anti-HER2 antibody-drug conjugate is in the range of from 7 to 8.
[85] The method of treatment according to any one of [67] to [83], wherein the average number of units of the drug-linker conjugated per antibody molecule in the anti-HER2 antibody-drug conjugate is in the range of from 7.5 to 8.
[86] The method of treatment according to any one of [67] to [85], wherein a dose per administration of the anti-HER2 antibody-drug conjugate is in a range of 5.4 mg/kg to 8 mg/kg.
[87] The method of treatment according to any one of [67] to [85], wherein a dose per administration of the anti-HER2 antibody-drug conjugate is 6.4 mg/kg.
[88] The method of treatment according to any one of [67] to [87], wherein the anti-HER2 antibody-drug conjugate is administered once every 3 weeks.
[89] An anti-HER2 antibody-drug conjugate for use in treating HER2-mutated cancer, in which a drug-linker represented by the following formula: 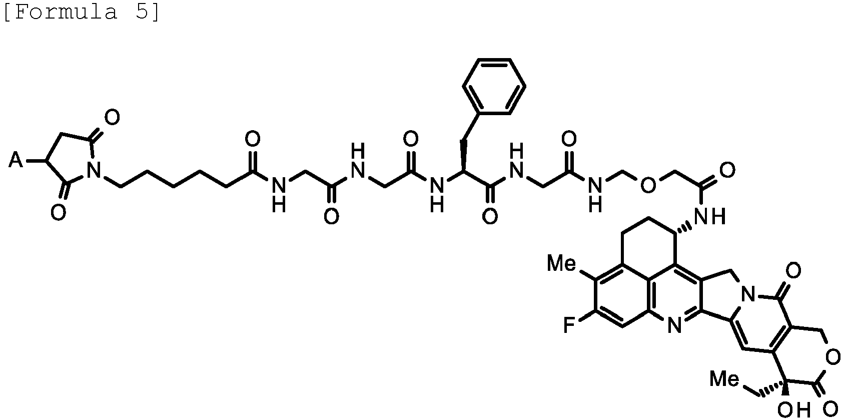 wherein A represents a connecting position to an anti-HER2 antibody;
   is conjugated to the anti-HER2 antibody via a thioether bond.
[90] The anti-HER2 antibody-drug conjugate according to [89], wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, I613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770_A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.
[91] The anti-HER2 antibody-drug conjugate according to [90], wherein the HER2 mutation in the HER2-mutated cancer is at least one selected from the group consisting of Y772_A775dup, G778_P780dup, G776delinsVC, L755S, V777L, and S310F.
[92] The anti-HER2 antibody-drug conjugate according to [89], wherein the HER2 mutation in the HER2-mutated cancer is an exon 20 insertion mutation.
[93] The anti-HER2 antibody-drug conjugate according to [92], wherein the exon 20 insertion mutation is at least one selected from the group consisting of Y772_A775dup, G778_P780dup, G776delinsVC, E770_A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.
[94] The anti-HER2 antibody-drug conjugate according to [93], wherein the exon 20 insertion mutation is at least one selected from the group consisting of Y772_A775dup, G778_P780dup, and G776delinsVC.
[95] The anti-HER2 antibody-drug conjugate according to [89], wherein the HER2 mutation in the HER2-mutated cancer is a single base pair substitution mutation at a transmembrane domain of a HER2 protein.
[96] The anti-HER2 antibody-drug conjugate according to [95], wherein the single base pair substitution mutation at the transmembrane domain of the HER2 protein is at least one selected from the group consisting of V659E, G660D, I654V, I655V, and I661V.
[97] The anti-HER2 antibody-drug conjugate according to [96], wherein the single base pair substitution mutation at the transmembrane domain of the HER2 protein is G660D.
[98] The anti-HER2 antibody-drug conjugate according to [89], wherein the HER2 mutation in the HER2-mutated cancer is a single base pair substitution mutation at an extracellular domain of a HER2 protein.
[99] The anti-HER2 antibody-drug conjugate according to [98], wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.
[100] The anti-HER2 antibody-drug conjugate according to [99], wherein the single base pair substitution mutation at the extracellular domain of the HER2 protein is S310F.
[101] The anti-HER2 antibody-drug conjugate according to any one of [89] to [100], wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.
[102] The anti-HER2 antibody-drug conjugate according to any one of [89] to [100], wherein the cancer is non-small cell lung cancer.
[103] The anti-HER2 antibody-drug conjugate according to [102], wherein the non-small cell lung cancer is unresectable and/or metastatic non-small cell lung cancer.
[104] The anti-HER2 antibody-drug conjugate according to any one of [89] to [103], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[105] The anti-HER2 antibody-drug conjugate according to any one of [89] to [103], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[106] The anti-HER2 antibody-drug conjugate according to any one of [89] to [105], wherein the average number of units of the drug-linker conjugated per antibody molecule in the anti-HER2 antibody-drug conjugate is in the range of from 7 to 8.
[107] The anti-HER2 antibody-drug conjugate according to any one of [89] to [105], wherein the average number of units of the drug-linker conjugated per antibody molecule in the anti-HER2 antibody-drug conjugate is in the range of from 7.5 to 8.
[108] The anti-HER2 antibody-drug conjugate according to any one of [89] to [107], wherein a dose per administration of the anti-HER2 antibody-drug conjugate is in a range of 5.4 mg/kg to 8 mg/kg.
[109] The anti-HER2 antibody-drug conjugate according to any one of [89] to [107], wherein a dose per administration of the anti-HER2 antibody-drug conjugate is 6.4 mg/kg.
[110] The anti-HER2 antibody-drug conjugate according to any one of [89] to [109], wherein the anti-HER2 antibody-drug conjugate is administered once every 3 weeks.
[111] An anti-HER2 antibody-drug conjugate for use in treating HER2-mutated cancer, represented by the following formula: 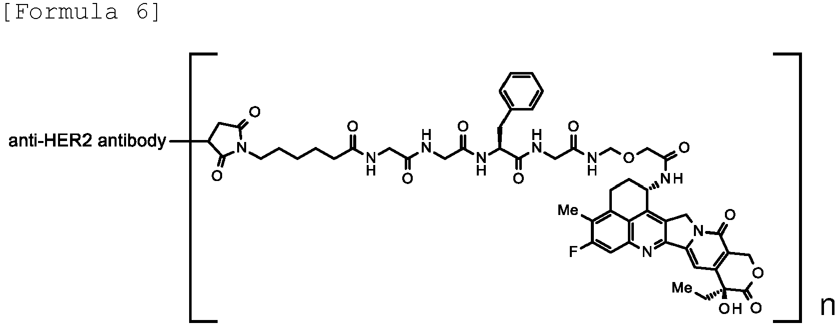 wherein the drug-linker is conjugated to the anti-HER2 antibody via a thioether bond, and n is the average number of units of the drug-linker conjugated per antibody molecule.
[112] The anti-HER2 antibody-drug conjugate according to [111], wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, I613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770 _A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.
[113] The anti-HER2 antibody-drug conjugate according to [112], wherein the HER2 mutation in the HER2-mutated cancer is at least one selected from the group consisting of Y772_A775dup, G778_P780dup, G776delinsVC, L755S, V777L, and S310F.
[114] The anti-HER2 antibody-drug conjugate according to [111], wherein the HER2 mutation in the HER2-mutated cancer is an exon 20 insertion mutation.
[115] The anti-HER2 antibody-drug conjugate according to [114], wherein the exon 20 insertion mutation is at least one selected from the group consisting of Y772_A775dup, G778_P780dup, G776delinsVC, E770_A771insAYVM, A771_Y772insYVMA, M774 _A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.
[116] The anti-HER2 antibody-drug conjugate according to [115], wherein the exon 20 insertion mutation is at least one selected from the group consisting of Y772_A775dup, G778_P780dup, and G776delinsVC.
[117] The anti-HER2 antibody-drug conjugate according to [111], wherein the HER2 mutation in the HER2-mutated cancer is a single base pair substitution mutation at a transmembrane domain of a HER2 protein.
[118] The anti-HER2 antibody-drug conjugate according to [117], wherein the single base pair substitution mutation at the transmembrane domain of the HER2 protein is at least one selected from the group consisting of V659E, G660D, I654V, I655V, and I661V.
[119] The anti-HER2 antibody-drug conjugate according to [118], wherein the single base pair substitution mutation at the transmembrane domain of the HER2 protein is G660D.
[120] The anti-HER2 antibody-drug conjugate according to [111], wherein the HER2 mutation in the HER2-mutated cancer is a single base pair substitution mutation at an extracellular domain of a HER2 protein.
[121] The anti-HER2 antibody-drug conjugate according to [120], wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.
[122] The anti-HER2 antibody-drug conjugate according to [121], wherein the single base pair substitution mutation at the extracellular domain of the HER2 protein is S310F.
[123] The anti-HER2 antibody-drug conjugate according to any one of [111] to [122], wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.
[124] The anti-HER2 antibody-drug conjugate according to any one of [111] to [122], wherein the cancer is non-small cell lung cancer.
[125] The anti-HER2 antibody-drug conjugate according to [124], wherein the non-small cell lung cancer is unresectable and/or metastatic non-small cell lung cancer.
[126] The anti-HER2 antibody-drug conjugate according to any one of [111] to [125], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[127] The anti-HER2 antibody-drug conjugate according to any one of [111] to [125], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[128] The anti-HER2 antibody-drug conjugate according to any one of [111] to [127], wherein the average number of units of the drug-linker conjugated per antibody molecule in the anti-HER2 antibody-drug conjugate is in the range of from 7 to 8.
[129] The anti-HER2 antibody-drug conjugate according to any one of [111] to [127], wherein the average number of units of the drug-linker conjugated per antibody molecule in the anti-HER2 antibody-drug conjugate is in the range of from 7.5 to 8.
[130] The anti-HER2 antibody-drug conjugate according to any one of [111] to [129], wherein a dose per administration of the anti-HER2 antibody-drug conjugate is in a range of 5.4 mg/kg to 8 mg/kg.
[131] The anti-HER2 antibody-drug conjugate according to any one of [111] to [129], wherein a dose per administration of the anti-HER2 antibody-drug conjugate is 6.4 mg/kg.
[132] The anti-HER2 antibody-drug conjugate according to any one of [111] to [131], wherein the anti-HER2 antibody-drug conjugate is administered once every 3 weeks.
[133] Use of an anti-HER2 antibody-drug conjugate for the manufacture of a medicament for treating HER2-mutated cancer, in which a drug-linker represented by the following formula: 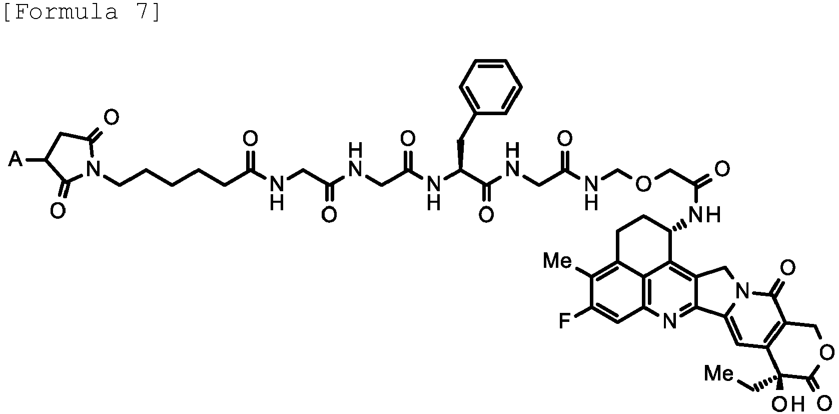 wherein A represents a connecting position to an anti-HER2 antibody;
   is conjugated to the anti-HER2 antibody via a thioether bond.
[134] The use according to [133], wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, I613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770 _A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.
[135] The use according to [134], wherein the HER2 mutation in the HER2-mutated cancer is at least one selected from the group consisting of Y772_A775dup, G778_P780dup, G776delinsVC, L755S, V777L, and S310F.
[136] The use according to [133], wherein the HER2 mutation in the HER2-mutated cancer is an exon 20 insertion mutation.
[137] The use according to [136], wherein the exon 20 insertion mutation is at least one selected from the group consisting of Y772_A775dup, G778_P780dup, G776delinsVC, E770 _A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.
[138] The use according to [137], wherein the exon 20 insertion mutation is at least one selected from the group consisting of Y772_A775dup, G778_P780dup, and G776delinsVC.
[139] The use according to [133], wherein the HER2 mutation in the HER2-mutated cancer is a single base pair substitution mutation at a transmembrane domain of a HER2 protein.
[140] The use according to [139], wherein the single base pair substitution mutation at the transmembrane domain of the HER2 protein is at least one selected from the group consisting of V659E, G660D, I654V, I655V, and I661V.
[141] The use according to [140], wherein the single base pair substitution mutation at the transmembrane domain of the HER2 protein is G660D.
[142] The use according to [133], wherein the HER2 mutation in the HER2-mutated cancer is a single base pair substitution mutation at an extracellular domain of a HER2 protein.
[143] The use according to [142], wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.
[144] The use according to [143], wherein the single base pair substitution mutation at the extracellular domain of the HER2 protein is S310F.
[145] The use according to any one of [133] to [144], wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.
[146] The use according to any one of [133] to [144], wherein the cancer is non-small cell lung cancer.
[147] The use according to [146], wherein the non-small cell lung cancer is unresectable and/or metastatic non-small cell lung cancer.
[148] The use according to any one of [133] to [147], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[149] The use according to any one of [133] to [147], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[150] The use according to any one of [133] to [149], wherein the average number of units of the drug-linker conjugated per antibody molecule in the anti-HER2 antibody-drug conjugate is in the range of from 7 to 8.
[151] The use according to any one of [133] to [149], wherein the average number of units of the drug-linker conjugated per antibody molecule in the anti-HER2 antibody-drug conjugate is in the range of from 7.5 to 8.
[152] The use according to any one of [133] to [151], wherein a dose per administration of the anti-HER2 antibody-drug conjugate is in a range of 5.4 mg/kg to 8 mg/kg.
[153] The use according to any one of [133] to [151], wherein a dose per administration of the anti-HER2 antibody-drug conjugate is 6.4 mg/kg.
[154] The use according to any one of [133] to [153], wherein the anti-HER2 antibody-drug conjugate is administered once every 3 weeks.
[155] Use of an anti-HER2 antibody-drug conjugate for the manufacture of a medicament for treating HER2-mutated cancer represented by the following formula: 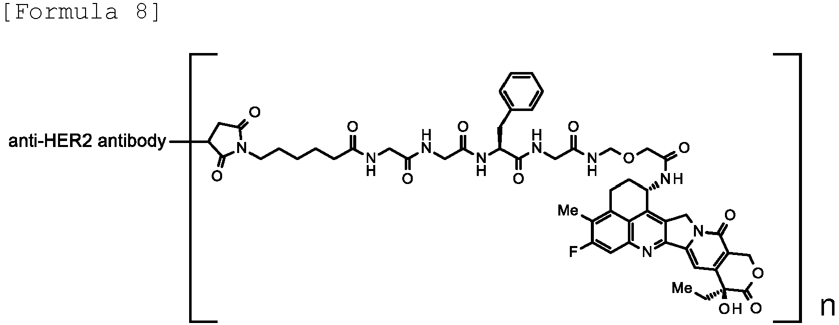 wherein the drug-linker is conjugated to the anti-HER2 antibody via a thioether bond, and n is the average number of units of the drug-linker conjugated per antibody molecule.
[156] The use according to [155], wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, I613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770 _A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.
[157] The use according to [156], wherein the HER2 mutation in the HER2-mutated cancer is at least one selected from the group consisting of Y772_A775dup, G778_P780dup, G776delinsVC, L755S, V777L, and S310F.
[158] The use according to [155], wherein the HER2 mutation in the HER2-mutated cancer is an exon 20 insertion mutation.
[159] The use according to [158], wherein the exon 20 insertion mutation is at least one selected from the group consisting of Y772_A775dup, G778_P780dup, G776delinsVC, E770 _A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.
[160] The use according to [159], wherein the exon 20 insertion mutation is at least one selected from the group consisting of Y772_A775dup, G778_P780dup, and G776delinsVC.
[161] The use according to [155], wherein the HER2 mutation in the HER2-mutated cancer is a single base pair substitution mutation at a transmembrane domain of a HER2 protein.
[162] The use according to [161], wherein the single base pair substitution mutation at the transmembrane domain of the HER2 protein is at least one selected from the group consisting of V659E, G660D, I654V, I655V, and I661V.
[163] The use according to [162], wherein the single base pair substitution mutation at the transmembrane domain of the HER2 protein is G660D.
[164] The use according to [155], wherein the HER2 mutation in the HER2-mutated cancer is a single base pair substitution mutation at an extracellular domain of a HER2 protein.
[165] The use according to [164], wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.
[166] The use according to [165], wherein the single base pair substitution mutation at the extracellular domain of the HER2 protein is S310F.
[167] The use according to any one of [155] to [166], wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.
[168] The use according to any one of [155] to [166], wherein the cancer is non-small cell lung cancer.
[169] The use according to [168], wherein the non-small cell lung cancer is unresectable and/or metastatic non-small cell lung cancer.
[170] The use according to any one of [155] to [169], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[171] The use according to any one of [155] to [169], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[172] The use according to any one of [155] to [171], wherein the average number of units of the drug-linker conjugated per antibody molecule in the anti-HER2 antibody-drug conjugate is in the range of from 7 to 8.
[173] The use according to any one of [155] to [171], wherein the average number of units of the drug-linker conjugated per antibody molecule in the anti-HER2 antibody-drug conjugate is in the range of from 7.5 to 8.
[174] The use according to any one of [155] to [173], wherein a dose per administration of the anti-HER2 antibody-drug conjugate is in a range of 5.4 mg/kg to 8 mg/kg.
[175] The use according to any one of [155] to [173], wherein a dose per administration of the anti-HER2 antibody-drug conjugate is 6.4 mg/kg.
[176] The use according to any one of [155] to [175], wherein the anti-HER2 antibody-drug conjugate is administered once every 3 weeks.

Further, the present invention can also be expressed as follows.
[1] A therapeutic agent for HER2 gene-mutated cancer comprising, as an active component, an anti-HER2 antibody-drug conjugate in which a drug-linker represented by the following formula: 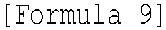 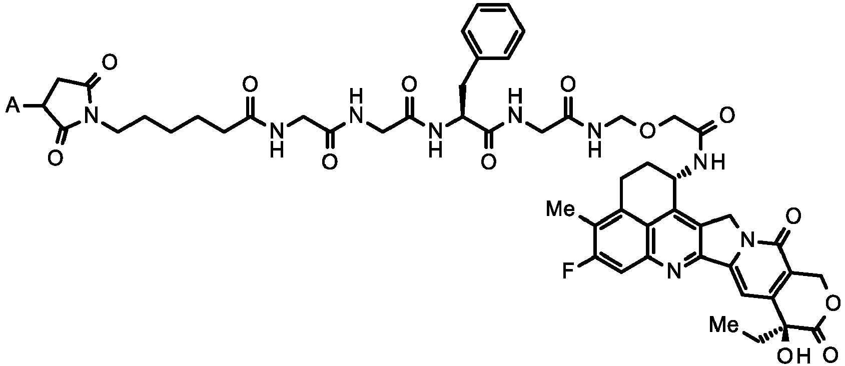 wherein A represents a connecting position to an anti-HER2 antibody;
   is conjugated to the anti-HER2 antibody via a thioether bond.
[2] The therapeutic agent according to [1], wherein the cancer is at least one selected from the group consisting of non-small cell lung cancer, breast cancer, gastric cancer, colorectal cancer, esophageal cancer, salivary gland cancer, gastroesophageal junction adenocarcinoma, bile duct cancer, Paget's disease, pancreatic cancer, ovarian cancer and uterine carcinosarcoma.
[3] The therapeutic agent according to [1], wherein the cancer is non-small cell lung cancer.
[4] The therapeutic agent according to any one of [1] to [3], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[5] The therapeutic agent according to any one of [1] to [3], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[6] The therapeutic agent according to any one of [1] to [5], wherein the average number of units of the drug-linker conjugated per antibody molecule in the anti-HER2 antibody-drug conjugate is in the range of from 7 to 8.
[7] The therapeutic agent according to any one of [1] to [5], wherein the average number of units of the drug-linker conjugated per antibody molecule in the anti-HER2 antibody-drug conjugate is in the range of from 7.5 to 8.
[8] The therapeutic agent according to any one of [1] to [7], wherein a dose per administration of the anti-HER2 antibody-drug conjugate is in a range of 5.4 mg/kg to 8 mg/kg.
[9] The therapeutic agent according to any one of [1] to [7], wherein a dose per administration of the anti-HER2 antibody-drug conjugate is 6.4 mg/kg.
[10] The therapeutic agent according to any one of [1] to [9], wherein the anti-HER2 antibody-drug conjugate is administered once every 3 weeks.
[11] A method of treatment for cancer, comprising administering an anti-HER2 antibody-drug conjugate in which a drug-linker represented by the following formula: 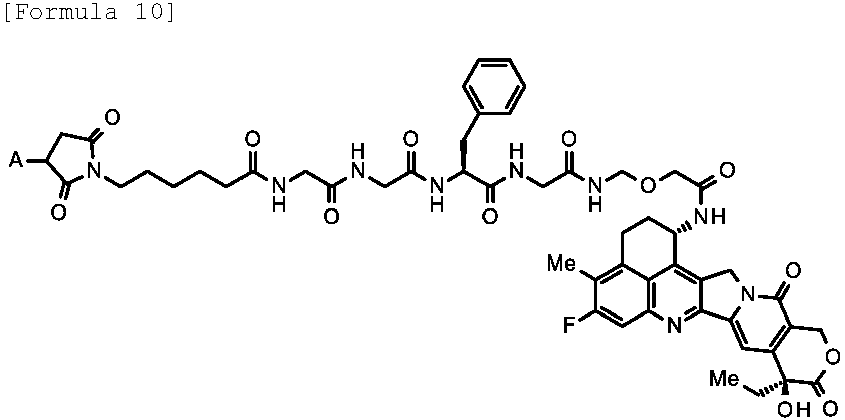 wherein A represents a connecting position to an anti-HER2 antibody;
   is conjugated to the anti-HER2 antibody via a thioether bond, to a subject determined to have HER2 gene mutated cancer.
[12] The method of treatment according to [11], wherein the cancer is at least one selected from the group consisting of non-small cell lung cancer, breast cancer, gastric cancer, colorectal cancer, esophageal cancer, salivary gland cancer, gastroesophageal junction adenocarcinoma, bile duct cancer, Paget's disease, pancreatic cancer, ovarian cancer and uterine carcinosarcoma.
[13] The method of treatment according to [11], wherein the cancer is non-small cell lung cancer.
[14] The method of treatment according to any one of [11] to [13], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[15] The method of treatment according to any one of [11] to [13], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[16] The method of treatment according to any one of [11] to [15], wherein the average number of units of the drug-linker conjugated per antibody molecule in the anti-HER2 antibody-drug conjugate is in the range of from 7 to 8.
[17] The method of treatment according to any one of [11] to [15], wherein the average number of units of the drug-linker conjugated per antibody molecule in the anti-HER2 antibody-drug conjugate is in the range of from 7.5 to 8.
[18] The method of treatment according to any one of [11] to [17], wherein a dose per administration of the anti-HER2 antibody-drug conjugate is in a range of 5.4 mg/kg to 8 mg/kg.
[19] The method of treatment according to any one of [11] to [17], wherein a dose per administration of the anti-HER2 antibody-drug conjugate is 6.4 mg/kg.
[20] The method of treatment according to any one of [11] to [19], wherein the anti-HER2 antibody-drug conjugate is administered once every 3 weeks.
[21] An anti-HER2 antibody-drug conjugate for use in treating HER2 gene-mutated cancer, in which a drug-linker represented by the following formula: 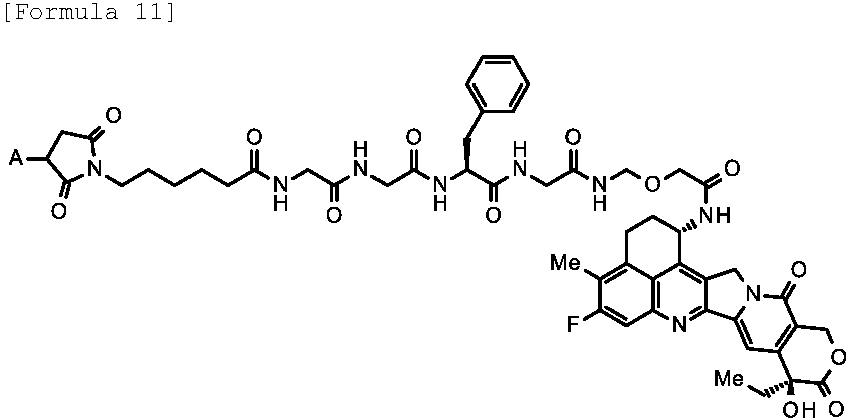 wherein A represents a connecting position to an anti-HER2 antibody;
   is conjugated to the anti-HER2 antibody via a thioether bond.
[22] The anti-HER2 antibody-drug conjugate according to [21], wherein the cancer is at least one selected from the group consisting of non-small cell lung cancer, breast cancer, gastric cancer, colorectal cancer, esophageal cancer, salivary gland cancer, gastroesophageal junction adenocarcinoma, bile duct cancer, Paget's disease, pancreatic cancer, ovarian cancer and uterine carcinosarcoma.
[23] The anti-HER2 antibody-drug conjugate according to [21], wherein the cancer is non-small cell lung cancer.
[24] The anti-HER2 antibody-drug conjugate according to any one of [21] to [23], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[25] The anti-HER2 antibody-drug conjugate according to any one of [21] to [23], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[26] The anti-HER2 antibody-drug conjugate according to any one of [21] to [25], wherein the average number of units of the drug-linker conjugated per antibody molecule is in the range of from 7 to 8.
[27] The anti-HER2 antibody-drug conjugate according to any one of [21] to [25], wherein the average number of units of the drug-linker conjugated per antibody molecule is in the range of from 7.5 to 8.
[28] The anti-HER2 antibody-drug conjugate according to any one of [21] to [27], wherein a dose per administration is in a range of 5.4 mg/kg to 8 mg/kg.
[29] The anti-HER2 antibody-drug conjugate according to any one of [21] to [27], wherein a dose per administration is 6.4 mg/kg.
[30] The anti-HER2 antibody-drug conjugate according to any one of [21] to [29], to be administered once every 3 weeks.
[31] Use of an anti-HER2 antibody-drug conjugate for the manufacture of a medicament for treating HER2 gene-mutated cancer, in which a drug-linker represented by the following formula: 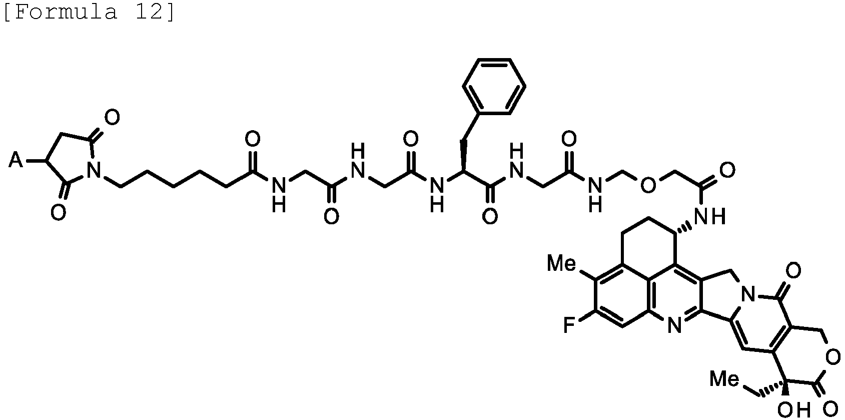 wherein A represents a connecting position to an anti-HER2 antibody;
   is conjugated to the anti-HER2 antibody via a thioether bond.
[32] The use according to [31], wherein the cancer is at least one selected from the group consisting of non-small cell lung cancer, breast cancer, gastric cancer, colorectal cancer, esophageal cancer, salivary gland cancer, gastroesophageal junction adenocarcinoma, bile duct cancer, Paget's disease, pancreatic cancer, ovarian cancer and uterine carcinosarcoma.
[33] The use according to [31], wherein the cancer is non-small cell lung cancer.
[34] The use according to any one of [31] to [33], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[35] The use according to any one of [31] to [33], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[36] The use according to any one of [31] to [35], wherein the average number of units of the drug-linker conjugated per antibody molecule in the anti-HER2 antibody-drug conjugate is in the range of from 7 to 8.
[37] The use according to any one of [31] to [35], wherein the average number of units of the drug-linker conjugated per antibody molecule in the anti-HER2 antibody-drug conjugate is in the range of from 7.5 to 8.
[38] The use according to any one of [31] to [37], wherein a dose per administration of the anti-HER2 antibody-drug conjugate is in a range of 5.4 mg/kg to 8 mg/kg.
[39] The use according to any one of [31] to [37], wherein a dose per administration of the anti-HER2 antibody-drug conjugate is 6.4 mg/kg.
[40] The use according to any one of [31] to [39], wherein the anti-HER2 antibody-drug conjugate is administered once every 3 weeks.

### Advantageous Effects of Invention

The present invention can provide a therapeutic agent for HER2-mutated cancer comprising a specific anti-HER2 antibody-drug conjugate and/or a method of treatment for HER2-mutated cancer comprising administering the specific anti-HER2 antibody-drug conjugate to a subject determined to have HER2-mutated cancer.

### Brief Description of Drawings

[Figure 1] Figure 1 shows an amino acid sequence (SEQ ID No: 1) of a heavy chain of a humanized anti-HER2 antibody.
[Figure 2] Figure 2 shows an amino acid sequence (SEQ ID No: 2) of a light chain of a humanized anti-HER2 antibody.

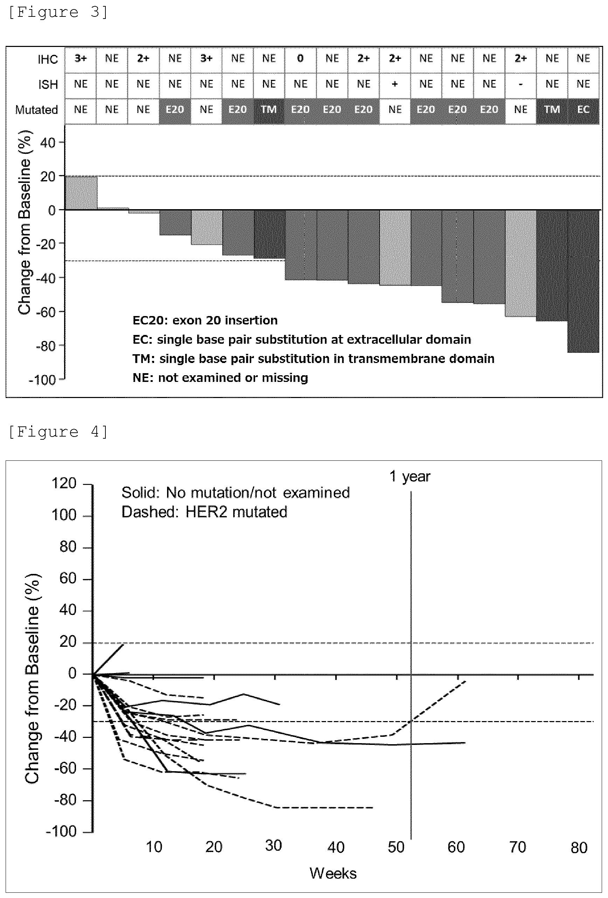

[Figure 3] Figure 3 shows maximum tumor shrinkages as the effectiveness of HER2-ADC (1) on subjects with non-small cell lung cancer determined to have HER2 expression or HER2 mutation. In the figure, "NE" represents subjects with HER2 mutation unmeasured or failed to be measured, "E20" represents subjects determined to have an Exon 20 insertion mutation in the HER2 protein, "TM" represents subjects determined to have a single base pair substitution mutation at a transmembrane domain of the HER2 protein, and "EC" represents subjects determined to have a single base pair substitution mutation at an extracellular domain of the HER2 protein.
[Figure 4] Figure 4 shows the time course of tumor shrinkages as the effectiveness of HER2-ADC (1) on subjects with non-small cell lung cancer determined to have HER2 expression or HER2 mutation.
[Figure 5] Figure 5 shows an amino acid sequence (SEQ ID No: 3) of HER2 protein.

### Description of Embodiments

Hereinafter, preferred modes for carrying out the present invention are described. The embodiments described below are given merely for illustrating one example of a typical embodiment of the present invention and are not intended to limit the scope of the present invention.

### [Definitions]

In the present invention, "HER2" is synonymous with human epidermal growth factor receptor 2 (which may also be referred to as neu or ErbB-2) and is a transmembrane receptor belonging to the epidermal growth factor receptor (EGFR) subfamily of receptor protein tyrosine kinases together with HER1 (EGFR or ErbB-1), HER3 (ErbB-3), and HER4 (ErbB-4). HER2 is known to play an important role in cell proliferation, differentiation, and survival in normal cells and tumor cells by being activated by autophosphorylation of intercellular tyrosine residues due to heterodimer formation with HER1, HER3, or HER4.

In the present invention, the term "HER2 protein" is used in the same meaning as HER2. The expression of the HER2 protein can be detected using a method well known to those skilled in the art, such as immunohistochemistry (IHC) .

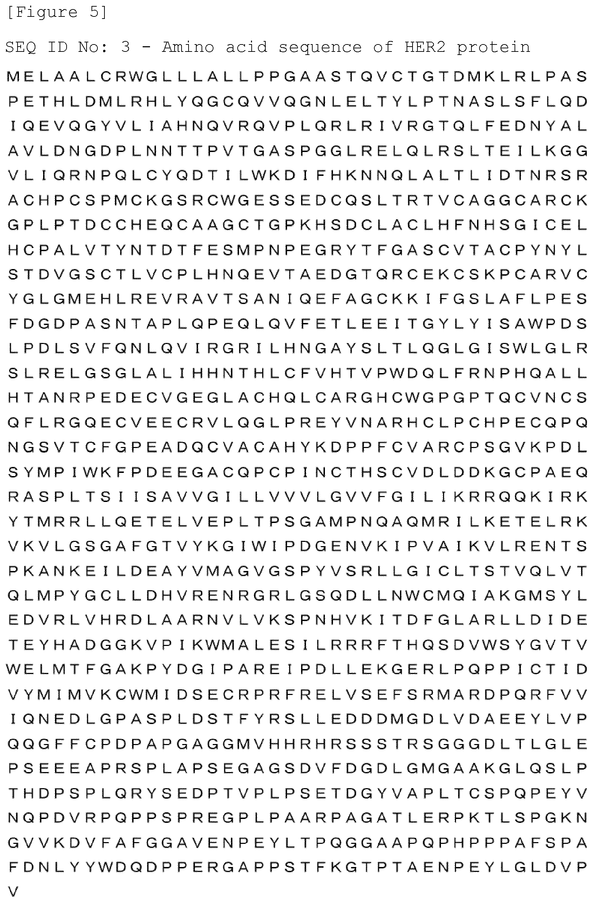

SEQ ID No: 3 (Figure 5) shows the amino acid sequence of the HER2 protein. In SEQ ID No: 3, the amino acid sequence consisting of amino acid residues 1 to 652 is referred to as the "extracellular domain of the HER2 protein", the amino acid sequence consisting of amino acid residues 653 to 675 is referred to as the "transmembrane domain of the HER2 protein", and the amino acid sequence consisting of amino acid residues 676 to 1255 is referred to as the "intercellular domain of the HER2 protein".

In the present invention, "HER2 gene" is synonymous with human epithelial cell growth factor receptor type 2-related cancer gene. The HER2 protein is one of the gene products of the HER2 gene.

SEQ ID No: 4 shows the nucleotide sequence of the HER2 gene (cDNA).

In the present invention, "HER2 mutation" means to have a mutation in the amino acid sequence of the HER2 protein.

In the present invention, "HER2-mutated cancer" means a cancer with a mutation in the amino acid sequence of the HER2 protein. Further, a cancer containing cancer cells with the HER2 mutation, even without the HER2 mutation in the entire tumor tissue, is included in the term HER2-mutated cancer.

In the present invention, "HER2 gene mutation" means to have a mutation in the HER2 gene.

In the present invention, "HER2 gene-mutated cancer" means a cancer with a mutation in the HER2 gene. Further, a cancer containing cancer cells with the HER2 gene mutation, even without the HER2 gene mutation in the entire tumor tissue, is included in the term HER2 gene-mutated cancer.

The HER2 gene mutation causes a mutation in the amino acid sequence of the HER2 protein that is a gene product, thereby causing the HER2 mutation.

Specific examples of the HER2 mutation can include a mutation that duplicates the amino acid sequence YVMA (tyrosine, valine, methionine, and alanine) at positions 772 to 775 of the HER2 protein (which may also be referred to as "Y772_A775dup" or "A775_G776insYVMA") (see Nature. 2004 Sep 30;431(7008):525-6, Cancer Res. 2005 Mar 1;65(5):1642-6, Cancer Res. 2005 Sep 1;65(17):7591-5, Int J Cancer. 2006 Dec 1;119(11):2586-91, Mol Cancer Res. 2008 Nov;6(11):1678-90, Nat Med. 2017 Jun;23(6):703-713, and Nature. 2018 Feb 8;554(7691):189-194, for example), a mutation that duplicates the amino acid sequence GSP (glycine, serine, and proline) at positions 778 to 780 of the HER2 protein (which may also be referred to as "G778_P780dup" or "P780_Y781insGSP") (see Cancer Res. 2005 Mar 1;65(5):1642-6, Pathobiology. 2008;75(1):2-8, Nature. 2012 May 16;486(7403):400-4, Clin Cancer Res. 2013 May 15;19(10):2668-76, Nature. 2016 Jun 2;534(7605):47-54, Cancer. 2016 Sep 1;122(17):2654-62, PLoS Med. 2016 Dec 27;13(12) :e1002201, and Nat Med. 2017 Jun;23(6):703-713, for example), a mutation that replaces G (glycine) at amino acid position 776 of the HER2 protein by VC (valine and cysteine) (which may also be referred to as "G776delinsVC" or "G776>VC") (see Cancer Res. 2005 Mar 1;65(5):1642-6, Cancer Sci. 2006 Aug;97(8):753-9, Cancer Genet Cytogenet. 2007 Mar;173(2):107-13, Clin Cancer Res. 2012 Sep 15;18(18):4910-8, and Nature. 2018 Feb 8;554(7691):189-194, for example), a mutation that replaces L (leucine) at amino acid position 755 of the HER2 protein by S (serine) (which may also be referred to as "L755S") (see Clin Cancer Res. 2006 Jan 1;12 (1) :57-61, Hum Mutat. 2008 Mar;29(3):441-50, Nature. 2012 Apr 4;486(7403):395-9, Breast Cancer Res Treat. 2012 Jul;134(2):561-7, Clin Cancer Res. 2012 Sep 15;18(18):4910-8, and Cancer Lett. 2013 Mar 1;330 (1) :33-40, for example), a mutation that replaces V (valine) at amino acid position 777 of the HER2 protein by L (leucine) (which may also be referred to as "V777L") (see Clin Cancer Res. 2006 Jan 1;12(1):57-61, Int J Cancer. 2006 Dec 1;119(11):2586-91, Nature. 2010 Aug 12;466(7308):869-73, Nature. 2012 Jun 10;486(7403):353-60, Cancer Cell. 2016 Feb 8;29(2):229-40, and Cancer. 2016 Sep 1;122(17):2654-62, for example), a mutation that replaces V (valine) at amino acid position 659 of the HER2 protein by E (glutamic acid) (which may also be referred to as "V659E") (see Cancer Discov. 2013 Nov;3(11):1238-44, J Natl Cancer Inst. 2014 Jan; 106 (1) :djt338, Nat Med. 2017 Jun;23(6):703-713, and Nature. 2018 Feb 8;554(7691):189-194, for example), a mutation that replaces G (glycine) at amino acid position 660 of the HER2 protein by D (aspartic acid) (which may also be referred to as "G660D"), (see Nat Genet. 2014 Dec;46(12):1264-6, Cancer Cell. 2016 Feb 8;29(2):229-40, and Cell Rep. 2016 Apr 26;15(4):857-865, for example), and a mutation that replaces S (serine) at amino acid position 310 of the HER2 protein by F (phenylalanine) (which may also be referred to as "S310F") (see Nature. 2008 Oct 23;455(7216):1069-75, Nature. 2011 Jun 29;474(7353):609-15, Nat Genet. 2011 Oct 30;43(12):1219-23, Nature. 2012 Apr 4;486(7403):395-9, Genome Res. 2012 Nov;22(11):2109-19, and Clin Cancer Res. 2013 May 15;19(10):2668-76, for example).

Further, other specific examples of the HER2 mutation can also include a mutation that replaces A (alanine) at amino acid position 20 of the HER2 protein by T (threonine) (which may also be referred to as "A20T") (see Nature 2010;466(7308):869-73, for example), a mutation that replaces A (alanine) at amino acid position 21 of the HER2 protein by S (serine) (which may also be referred to as "A21S") (see Nature medicine 2017;23(6):703-713, for example), a mutation that replaces R (arginine) at amino acid position 143 of the HER2 protein by Q (glutamine) (which may also be referred to as "R143Q") (see Nature communications 2015;6:10131, and Cancer biology & therapy 2014;15(9):1239-47, for example), a mutation that replaces K (lysine) at amino acid position 200 of the HER2 protein by N (asparagine) (which may also be referred to as "K200N") (see Nature medicine 2017;23(6):703-713, for example), a mutation that replaces A (alanine) at amino acid position 242 of the HER2 protein by V (valine) (which may also be referred to as "A242V") (see Nature medicine 2017;23(6):703-713, for example), a mutation that replaces D (aspartic acid) at amino acid position 277 of the HER2 protein by Y (tyrosine) (which may also be referred to as "D277Y") (see Nature medicine 2017;23(6):703-713, and Nature genetics 2013;45(12):1459-63, for example), a mutation that replaces A (alanine) at amino acid position 293 of the HER2 protein by P (proline) (which may also be referred to as "A293P") (see Nature medicine 2017;23(6):703-713, for example), a mutation that replaces N (asparagine) at amino acid position 302 of the HER2 protein by K (lysine) (which may also be referred to as "N302K") (see Nature medicine 2017;23(6):703-713, for example), a mutation that replaces V (valine) at amino acid position 308 of the HER2 protein by M (methionine) (which may also be referred to as "V308M") (see Cell Rep. 2016 Apr 26;15(4):857-865, for example), a mutation that replaces S (serine) at amino acid position 310 of the HER2 protein by Y (tyrosine) (which may also be referred to as "S310Y") (see Nature genetics 2014;46(8):872-6, Nature 2016;534(7605):47-54, and Nature medicine 2017;23(6):703-713, for example), a mutation that replaces N (asparagine) at amino acid position 319 of the HER2 protein by Y (tyrosine) (which may also be referred to as "N319Y") (see Cell. 2018 May 3;173(4):864-878. e29, for example), a mutation that replaces S (serine) at amino acid position 335 of the HER2 protein by C (cysteine) (which may also be referred to as "S335C") (see Nature medicine 2017;23(6):703-713, for example), a mutation that replaces R (arginine) at amino acid position 340 of the HER2 protein by P (proline) (which may also be referred to as "R340P") (see Nature medicine 2017;23(6):703-713, for example), a mutation that replaces S (serine) at amino acid position 418 of the HER2 protein by T (threonine) (which may also be referred to as "S418T") (see Cell 2012;150(6):1107-20, for example), a mutation that replaces W (tryptophan) at amino acid position 452 of the HER2 protein by C (cysteine) (which may also be referred to as "W452C") (see Cell. 2018 May 3;173(4):864-878. e29, for example), a mutation that replaces V (valine) at amino acid position 541 of the HER2 protein by M (methionine) (which may also be referred to as "V541M") (see Cell. 2018 May 3;173(4):864-878. e29, for example), a mutation that replaces I (isoleucine) at amino acid position 613 of the HER2 protein by V (valine) (which may also be referred to as "I613V") (see Scientific reports 2016;6:31628, for example), a mutation that replaces P (proline) at amino acid position 627 of the HER2 protein by H (histidine) (which may also be referred to as "P627H") (see PloS one 2016;11(4):e0154133, for example), a mutation that replaces A (alanine) at amino acid position 644 of the HER2 protein by V (valine) (which may also be referred to as "A644V") (see Nat Genet. 2012 Oct;44(10):1104-10, and Nature 2015;524(7563):47-53, for example), a mutation that replaces R (arginine) at amino acid position 647 of the HER2 protein by G (glycine) (which may also be referred to as "R647G") (see Cell. 2018 May 3;173(4):864-878. e29, for example), a mutation that replaces I (isoleucine) at amino acid position 654 of the HER2 protein by V (valine) (which may also be referred to as "I654V") (see Br J Cancer. 2017 Jun 27;117(1) :136-143, and Cell. 2018 May 3;173(4):864-878. e29, for example), a mutation that replaces I (isoleucine) at amino acid position 655 of the HER2 protein by V (valine) (which may also be referred to as "I655V") (see Oncotarget 2017;8(40):68026-68037, Nature communications 2015;6:10131, and Nature genetics 2014;46(6):595-600, for example), a mutation that replaces I (isoleucine) at amino acid position 661 of the HER2 protein by V (valine) (which may also be referred to as "I661V") (see Nature medicine 2017;23(6):703-713, for example), a mutation that replaces R (arginine) at amino acid position 678 of the HER2 protein by Q (glutamine) (which may also be referred to as "R678Q") (see Nature 2018;554(7691):189-194, for example), a mutation that replaces Q (glutamine) at amino acid position 680 of the HER2 protein by H (histidine) (which may also be referred to as "Q680H") (see Nature medicine 2017;23(6):703-713, for example), a mutation that replaces V (valine) at amino acid position 697 of the HER2 protein by L (leucine) (which may also be referred to as "V697L") (see Nature 2018;554(7691):189-194, Nature medicine 2017;23(6):703-713, and PLoS medicine 2016;13(12):e1002162, for example), a mutation that replaces G (glycine) at amino acid position 704 of the HER2 protein by R (arginine) (which may also be referred to as "G704R") (see Lung 2016;194(1):125-35, for example), a mutation that replaces Q (glutamine) at amino acid position 709 of the HER2 protein by L (leucine) (which may also be referred to as "Q709L") (see Nature medicine 2017;23(6):703-713, for example), a mutation that replaces Q (glutamine) at amino acid position 711 of the HER2 protein by H (histidine) (which may also be referred to as "Q711H") (see Nature medicine 2017;23(6):703-713, for example), a mutation that replaces G (glycine) at amino acid position 727 of the HER2 protein by A (alanine) (which may also be referred to as "G727A") (see Nature medicine 2017;23(6):703-713, for example), a mutation that replaces T (threonine) at amino acid position 733 of the HER2 protein by I (isoleucine) (which may also be referred to as "T733I") (see Nature 2018;554(7691):189-194, for example), a mutation that replaces E (glutamic acid) at amino acid position 744 of the HER2 protein by G (glycine) (which may also be referred to as "E744G") (see Cancer research 2007;67(12):5667-72, for example), a mutation that replaces N (asparagine) at amino acid position 745 of the HER2 protein by D (aspartic acid) (which may also be referred to as "N745D") (see Cancer research 2007;67(12):5667-72, for example), a mutation that replaces L (leucine) at amino acid position 755 of the HER2 protein by P (proline) (which may also be referred to as "L755P") (see Oncotarget 2016;7(28):44322-44329, Nature 2004;431(7008):525-6, and Nature medicine 2017;23(6):703-713, for example), a mutation that replaces L (leucine) at amino acid position 755 of the HER2 protein by A (alanine) (which may also be referred to as "L755A") (see Nature 2018;554(7691):189-194, and Nature medicine 2017;23(6):703-713, for example), a mutation that replaces L (leucine) at amino acid position 755 of the HER2 protein by F (phenylalanine) (which may also be referred to as "L755F") (see Clin Cancer Res. 2015 Aug 15;21(16):3631-9, for example), a mutation that replaces S (serine) at amino acid position 760 of the HER2 protein by F (phenylalanine) (which may also be referred to as "S760F") (see Clin Cancer Res. 2006 Apr 15;12(8):2538-44, for example), a mutation that replaces D (aspartic acid) at amino acid position 769 of the HER2 protein by H (histidine) (which may also be referred to as "D769H") (see Cancer 2016; 122(17):2654-62, Nature medicine 2017;23(6):703-713, and Nature 2018;554(7691):189-194, for example), a mutation that replaces D (aspartic acid) at amino acid position 769 of the HER2 protein by N (asparagine) (which may also be referred to as "D769N") (see Nature 2018;554(7691):189-194, for example), and a mutation that replaces D (aspartic acid) at amino acid position 769 of the HER2 protein by Y (tyrosine) (which may also be referred to as "D769Y") (see Nature 2018;554(7691):189-194, for example).

Further, other specific examples of the HER2 mutation can also include a mutation that inserts AYVM (alanine, tyrosine, valine, and methionine) between E (glutamic acid) at amino acid position 770 and A (alanine) at amino acid position 771 of the HER2 protein (which may also be referred to as "E770_A771insAYVM") (see Nature medicine 2017;23(6):703-713, and Nature 2018;554(7691):189-194, for example), a mutation that inserts YVMA (tyrosine, valine, methionine, and alanine) between A (alanine) at amino acid position 771 and Y (tyrosine) at amino acid position 772 of the HER2 protein (which may also be referred to as "A771_Y772insYVMA") (see Nature 2018;554(7691):189-194, for example), a mutation that inserts AYVM (alanine, tyrosine, valine, and methionine) between M (methionine) at amino acid position 774 and A (alanine) at amino acid position 775 of the HER2 protein (which may also be referred to as "M774_A775insAYVM") (see J Thorac Oncol. 2013 Feb;8(2):el9-20, and The American journal of surgical pathology 2006;30(10):1309-15, for example), a mutation that inserts YVMA (tyrosine, valine, methionine, and alanine) between A (alanine) at amino acid position 775 and G (glycine) at amino acid position 776 of the HER2 protein (which may also be referred to as "A775_G776insYVMA") (see Clin Cancer Res. 2013 May 15;19(10):2668-76, Cancer 2016;122(17):2654-62, and European urology 2016;70(2):348-57, for example), a mutation that replaces A (alanine) at amino acid position 775 of the HER2 protein by G (glycine) (which may also be referred to as "A775G") (see Anticancer research 2013;33(11):5127-33, for example), a mutation that replaces G (glycine) at amino acid position 776 of the HER2 protein by LC (leucine and cysteine) (which may also be referred to as "G776delinsLC" or "G776>LC") (see Cancer research 2005;65(5):1642-6, for example), a mutation that replaces G (glycine) at amino acid position 776 of the HER2 protein by C (cysteine) (which may also be referred to as "G776C") (see Clin Cancer Res. 2012 Sep 15;18(18):4910-8, for example), a mutation that replaces G (glycine) at amino acid position 776 of the HER2 protein by AVGC (alanine, valine, glycine, and cysteine) (which may also be referred to as "G776delinsAVGC" or "G776>AVGC") (see Nature 2018;554(7691):189-194, and Nature medicine 2017;23(6):703-713, for example), a mutation that replaces G (glycine) at amino acid position 776 of the HER2 protein by VV (valine and valine) (which may also be referred to as "G776delinsVV" or "G776>VV") (see Nature medicine 2017;23(6):703-713, for example), a mutation that inserts L (leucine) between G (glycine) at amino acid position 776 and V (valine) at amino acid position 777 of the HER2 protein (which may also be referred to as "G776_V777insL") (see Oncotarget 2016;7(20):29761-9, for example), a mutation that replaces G (glycine) at amino acid position 776 of the HER2 protein by L (leucine) (which may also be referred to as "G776L") (see Lung Cancer. 2012 Apr;76(1):123-7, for example), a mutation that inserts VC (valine and cysteine) between G (glycine) at amino acid position 776 and V (valine) at amino acid position 777 of the HER2 protein (which may also be referred to as "G776_V777insVC") (see J Clin Oncol. 2013 Jun 1;31(16):1997-2003, for example), a mutation that inserts VGC (valine, glycine, and cysteine) inserted between G (glycine) at amino acid position 776 and V (valine) at amino acid position 777 of the HER2 protein (which may also be referred to as "G776_V777insVGC") (see Nature 2018;554(7691):189-194, for example), a mutation that inserts CG (cysteine and glycine) between V (valine) at amino acid position 777 and G (glycine) at amino acid position 778 of the HER2 protein (which may also be referred to as "V777_G778insCG") (see Clin Cancer Res. 2012 Sep 15;18(18):4910-8, for example), a mutation that inserts G (glycine) between V (valine) at amino acid position 777 and G (glycine) at amino acid position 778 of the HER2 protein (which may also be referred to as "V777_G778insG") (see Nature 2018;554(7691):189-194, for example), a mutation that inserts G (glycine) between G (glycine) at amino acid position 778 and S (serine) at amino acid position 779 of the HER2 protein (which may also be referred to as "G778_S779insG") (see Nature 2018;554(7691):189-194, for example), a mutation that replaces S (serine) at amino acid position 779 of the HER2 protein by P (proline) (which may also be referred to as "S779P") (see Virchows Arch. 2016 Jun;468(6):651-62, for example), a mutation that inserts VGS (valine, glycine, and serine) between S (serine) at amino acid position 779 and P (proline) at amino acid position 780 of the HER2 protein (which may also be referred to as "S779_P780insVGS") (see Nature 2004;431(7008):525-6, and J Thorac Oncol. 2009 Jan;4(1):5-11, for example), a mutation that inserts GSP (glycine, serine, and proline) between P (proline) at amino acid position 780 and Y (tyrosine) at amino acid position 781 of the HER2 protein (which may also be referred to as "P780_Y781insGSP") (see Nature medicine 2017;23(6):703-713, for example), a mutation that replaces R (arginine) at amino acid position 784 of the HER2 protein by C (cysteine) (which may also be referred to as "R784C") (see Gynecol Oncol. 2018 Feb;148(2):311-316, Nature genetics 2014;46(12):1264-6, and Cell Rep. 2016 Apr 26;15(4):857-865, for example), a mutation that replaces R (arginine) at amino acid position 784 of the HER2 protein by H (histidine) (which may also be referred to as "R784H") (see Cell Rep. 2016 Apr 26;15(4):857-865, Virchows Arch. 2016 Jun;468(6):651-62, and Eur J Cancer. 2014 Jul;50(10):1740-1746, for example), a mutation that replaces L (leucine) at amino acid position 785 of the HER2 protein by R (arginine) (which may also be referred to as "L785R") (see Br J Cancer. 2015 Dec 22;113(12):1704-11, for example), a mutation that replaces L (leucine) at amino acid position 786 of the HER2 protein by V (valine) (which may also be referred to as "L786V") (see Nature 2018;554(7691):189-194, for example), a mutation that replaces T (threonine) at amino acid position 791 of the HER2 protein by I (isoleucine) (which may also be referred to as "T791I") (see Cancer research 2007;67(12):5667-72, for example), a mutation that replaces G (glycine) at amino acid position 804 of the HER2 protein by S (serine) (which may also be referred to as "G804S") (see Clin Cancer Res. 2006 Apr 15;12(8):2538-44, for example), a mutation that replaces L (leucine) at amino acid position 807 of the HER2 protein by F (phenylalanine) (which may also be referred to as "L807F") (see Eur J Cancer. 2015 Sep;51(13):1803-11, for example), a mutation that replaces S (serine) at amino acid position 819 of the HER2 protein by F (phenylalanine) (which may also be referred to as "S819F") (see PLoS medicine 2016;13(12):e1002162, and Cancer cell 2015;27(3):327-41, for example), a mutation that replaces I (isoleucine) at amino acid position 829 of the HER2 protein by T (threonine) (which may also be referred to as "I829T") (see Clin Cancer Res. 2006 Apr 15;12(8):2538-44, for example), a mutation that replaces V (valine) at amino acid position 842 of the HER2 protein by I (isoleucine) (which may also be referred to as "V842I") (see Nature 2018;554(7691):189-194, for example), a mutation that replaces L (leucine) at amino acid position 846 of the HER2 protein by F (phenylalanine) (which may also be referred to as "L846F") (see Oncotarget. 2016 Sep 20;7(38):61755-61763, for example), a mutation that replaces T (threonine) at amino acid position 862 of the HER2 protein by I (isoleucine) (which may also be referred to as "T862I") (see Nature 2018;554(7691):189-194, for example), a mutation that replaces R (arginine) at amino acid position 868 of the HER2 protein by W (tryptophan) (which may also be referred to as "R868W") (see Nature 2012;487(7407):330-7, and Br J Cancer. 2015 Dec 22;113(12):1704-11, for example), a mutation that replaces L (leucine) at amino acid position 869 of the HER2 protein by R (arginine) (which may also be referred to as "L869R") (see Nature medicine 2017;23(6):703-713, Nature 2018;554(7691):189-194, and PLoS medicine 2016;13(12):e1002201, for example), a mutation that replaces T (threonine) at amino acid position 875 of the HER2 protein by I (isoleucine) (which may also be referred to as "T875I") (see Br J Cancer. 2015 Dec 22;113(12):1704-11, for example), a mutation that deletes W (tryptophan) at amino acid position 906 of the HER2 protein (which may also be referred to as "W906*") (see Nature 2008;455(7216):1069-75, for example), a mutation that replaces T (threonine) at amino acid position 917 of the HER2 protein by S (serine) (which may also be referred to as "T917S") (see Carcinogenesis. 2012 Jul;33(7):1270-6, for example), a mutation that deletes Q (glutamine) at amino acid position 943 of the HER2 protein (which may also be referred to as "Q943*") (see Cell 2012;150(6):1107-20, for example), a mutation that deletes S (serine) at amino acid position 1007 of the HER2 protein (which may also be referred to as "S1007*") (see Cell. 2018 May 3;173(4):864-878. e29, for example), and a mutation that replaces S (serine) at amino acid position 1151 of the HER2 protein by L (leucine) (which may also be referred to as "S1151L") (see Nature medicine 2017;23(6):703-713, for example).

The HER2 mutation in the present invention is not specifically limited, as long as the amino acid sequence of the HER2 protein has a mutation, but specific examples thereof can include at least one selected from the group consisting of the aforementioned HER2 mutations, and preferably, at least one selected from the group consisting of Y772_A775dup, G778_P780dup, G776delinsVC, L755S, V777L, and S310F.

In the present invention, "Exon 20 insertion mutation" means a HER2 mutation caused by a base pair insertion into exon 20 of the HER2 gene. Exon 20 of the HER2 gene is represented by a nucleotide sequence of nucleotides 2308 to 2493 of SEQ ID No: 4, and the HER2 protein encoded thereby is represented by an amino acid sequence consisting of amino acid residues 770 to 831 of SEQ ID No: 3.

The Exon 20 insertion mutation in the present invention is not specifically limited, as long as it is a HER2 mutation caused by a base pair insertion into exon 20 of the HER2 gene, but examples thereof can include at least one selected from the group consisting of Y772_A775dup, G778_P780dup, G776delinsVC, E770_A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP, and preferably, at least one selected from the group consisting of Y772_A775dup, G778_P780dup, and G776delinsVC.

In the present invention, "single base pair substitution mutation" means a HER2 mutation caused by substitution of one base pair of the HER2 gene with another base pair.

The single base pair substitution mutation in the present invention is not specifically limited, as long as it is a HER2 mutation caused by substitution of one base pair of the HER2 gene with another base pair, but examples thereof can include at least one selected from the group consisting of L755S, V777L, V659E, G660D, S310F, A20T, A21S, R143Q, K200N, A242V, D277Y, A293P, N302K, V308M, S310Y, N319Y, S335C, R340P, S418T, W452C, V541M, 1613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, G776C, G776L, S779P, R784C, R784H, L785R, L786V, T791I, G804S, L807F, S819F, I829T, V842I, L846F, T862I, R868W, L869R, T875I, W906*, T917S, Q943*, S1007*, and S1151L, and preferably, at least one selected from the group consisting of L755S, V777L, V659E, G660D, and S310F.

In the present invention, "single base pair substitution mutation at a transmembrane domain of a HER2 protein" means a HER2 mutation caused at a transmembrane domain of the HER2 protein by substitution of one base pair of the HER2 gene with another base pair.

The single base pair substitution mutation at the transmembrane domain of the HER2 protein in the present invention is not specifically limited, as long as it is a HER2 mutation caused at a transmembrane domain of the HER2 protein by substitution of one base pair of the HER2 gene with another base pair, but examples thereof can include at least one selected from the group consisting of V659E, G660D, I654V, I655V, and I661V, and preferably, G660D.

In the present invention, "single base pair substitution mutation at an extracellular domain of a HER2 protein" means a HER2 mutation caused at an extracellular domain of the HER2 protein by substitution of one base pair of the HER2 gene with another base pair.

The single base pair substitution mutation at the extracellular domain of the HER2 protein in the present invention is not specifically limited, as long as it is a HER2 mutation caused at an extracellular domain of the HER2 protein by substitution of one base pair of the HER2 gene with another base pair, but examples thereof can include at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G, and preferably, S310F.

The therapeutic agent and/or the method of treatment of the present invention can be preferably used for HER2-mutated cancer determined to have at least one of the aforementioned mutations, but there is no limitation to these mutations as long as the cancer has a mutation in HER2.

The presence or absence of a HER2 mutation can be determined, for example, by collecting tumor tissue from a subject with cancer and subjecting a formalin-fixed paraffin-embedded specimen (FFPE) to real-time quantitative PCR (qRT-PCR), or microarray analysis.

Further, the presence or absence of a HER2 mutation can also be determined by collecting cell-free circulating tumor DNA (ctDNA) from a subject with cancer and subjecting it to next generation sequence (NGS) and so on (see J Clin Oncol 2013;31:1997-2003, Clin CancerRes 2012;18:4910-8, J Thorac Oncol 2012;7:85-9, Lung Cancer 2011;74:139-44, Cancer Res2005;65:1642-6, Cancer Sci 2006;97:753-9, ESMO Open 2017;2:e000279, and Annals ofOncology 26:1421-1427,2015, for example).

In the present invention, the term "HER2 mutation" is used in the same meaning as HER2 gene mutation.

In the present invention, "anti-HER2 antibody" means an antibody that specifically binds to HER2, preferably, has an activity of binding to HER2 and thereby being internalized into HER2-expressing cells, in other words, an antibody having an activity of binding to HER2 and thereafter moving into HER2-expressing cells.

### [Anti-HER2 Antibody-Drug Conjugate]

The anti-HER2 antibody-drug conjugate used in the present invention is an anti-HER2 antibody-drug conjugate in which a drug-linker represented by the following formula: 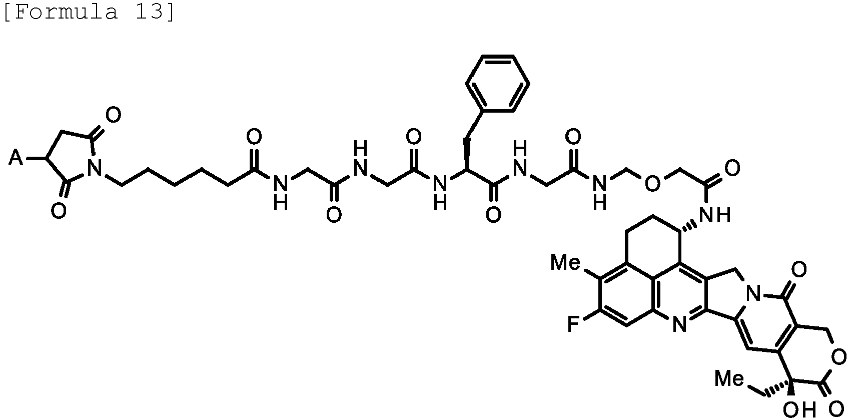 wherein A represents a connecting position to an antibody;
is conjugated to the antibody via a thioether bond.

In the present invention, a partial structure consisting of the linker and the drug of the anti-HER2 antibody-drug conjugate is referred to as "drug-linker". The drug-linker is connected to a thiol group (in other words, the sulfur atom of a cysteine residue) formed at an interchain disulfide bond site (two sites between heavy chains and two sites between a heavy chain and a light chain) of the antibody.

The drug-linker of the present invention includes exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4' :6,7]indolizino[1,2-b]quinoline-10,13-dione, (also expressed as chemical name: (1S,9S)-1-amino-9-ethyl-5-fluoro-2,3-dihydro-9-hydroxy-4-methyl-1H,12H-benzo[de]pyrano[3',4' :6,7]indolizino[1,2-b]quinoline-10,13(9H,15H)-dione)), which is a topoisomerase I inhibitor, as a component. Exatecan is a camptothecin derivative represented by the following formula: 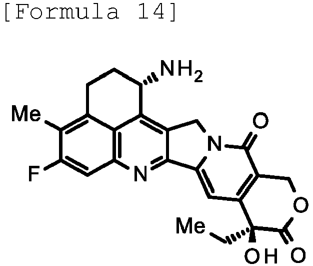 and having an antitumor effect.

The anti-HER2 antibody-drug conjugate used in the present invention can also be represented by the following formula.

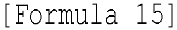

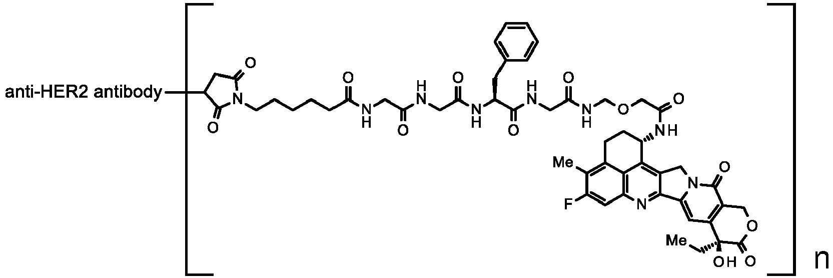

wherein the drug-linker is conjugated to the anti-HER2 antibody via a thioether bond. The meaning of n is the same as that of what is called the average number of conjugated drug molecules (DAR; Drug-to-Antibody Ratio), and indicates the average number of units of the drug-linker conjugated per antibody molecule.

The average number of units of the drug-linker conjugated per antibody molecule in the anti-HER2 antibody-drug conjugate used in the present invention is preferably 2 to 8, more preferably 3 to 8, even more preferably 7 to 8, even more preferably 7.5 to 8, even more preferably about 8.

After migrating into cancer cells, the anti-HER2 antibody-drug conjugate used in the present invention is cleaved at the linker moiety and releases the compound represented by the following formula:

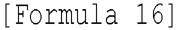

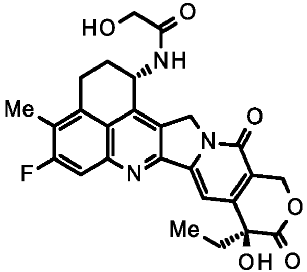

The aforementioned compound is inferred to be the original source of the antitumor activity of the anti-HER2 antibody-drug conjugate used in the present invention, and has been confirmed to have a topoisomerase I inhibitory effect (Ogitani Y. et al., Clinical Cancer Research, 2016, Oct15;22(20):5097-5108, Epub 2016 Mar 29).

The anti-HER2 antibody-drug conjugate used in the present invention is also known to have a bystander effect (Ogitani Y. et al., Cancer Science (2016)107,1039-1046). The bystander effect is exerted through a process in which the anti-HER2 antibody-drug conjugate used in the present invention is internalized in cancer cells expressing the target and the aforementioned compound then exerts an antitumor effect also on cancer cells which are present therearound and not expressing the target.

The anti-HER2 antibody-drug conjugate used in the present invention can be produced with reference to the description in International Publication No. WO 2015/115091 and so on.

### [Production of Anti-HER2 Antibody]

HER2 protein used in the present invention can be directly purified from HER2-expressing cells of humans or non-human mammals (such as rats and mice) or prepared using cell membrane fractions of such cells, or can be obtained by synthesizing HER2 in vitro or producing HER2 in host cells by genetic engineering. In genetic engineering, the protein can be obtained specifically by incorporating HER2 cDNA into an expressible vector and thereafter synthesizing HER2 in a solution containing enzymes, substrates, and energy materials required for transcription and translation, or transforming other prokaryotic or eukaryotic host cells to express HER2. Further, it is also possible to use the HER2-expressing cells by genetic engineering or the cell line expressing HER2 as the HER2 protein.

The HER2 protein used in the present invention can be directly purified from human HER2-expressing cells, can be prepared using cell membrane fractions of such cells as the HER2 protein, when used as an antigen, or can be obtained by synthesizing HER2 in vitro or producing HER2 in host cells by genetic engineering. In genetic engineering, HER2 can be synthesized specifically by incorporating HER2 cDNA into an expressible vector and thereafter incubating the vector in a solution containing enzymes, substrates, and energy materials required for transcription and translation. Alternatively, the protein can be obtained by transforming other prokaryotic or eukaryotic host cells using the vector to express HER2. Further, it is also possible to use the HER2-expressing cells by genetic engineering or the cell line expressing HER2 as HER2 protein antigen.

The DNA sequence and the amino acid sequence of HER2 are published on public databases and can be referred to, for example, by accession numbers such as M11730 (Genbank) and NP_004439.2 (NCBI).

Further, a protein consisting of an amino acid sequence with one or several amino acids substituted, deleted, and/or added in the amino acid sequence of HER2 and having a biological activity equivalent to that of the protein is also included in HER2.

Human HER2 protein is composed of a signal sequence consisting of N-terminal 22 amino acid residues, an extracellular domain consisting of 630 amino acid residues, a cell transmembrane domain consisting of 23 amino acid residues, and an intercellular domain consisting of 580 amino acid residues.

The anti-HER2 antibody used in the present invention can be obtained by known methods. For example, the antibody can be obtained using a method conventionally carried out in the art, which involves immunizing animals with any polypeptide selected from HER2 serving as an antigen or the amino acid sequence of HER2, collecting an antibody produced in vivo, and purifying the antibody. The origin of the antigen is not limited to humans, and animals may be immunized with an antigen derived from a non-human animal such as a mouse, a rat and the like. In this case, the cross-reactivity of antibodies binding to the obtained heterologous antigen with human antigens can be tested to screen for an anti-HER2 antibody applicable to a human disease.

Alternatively, antibody-producing cells which produce antibodies against the antigen are fused with myeloma cells according to a method known in the art (for example, Kohler and Milstein, Nature (1975) 256, p.495-497; Kennet, R. ed., Monoclonal Antibodies, p.365-367, Plenum Press, N.Y. (1980)), to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The anti-HER2 antibody used in the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

As the chimeric antibody, an antibody in which antibody variable and constant regions are derived from different species, for example, a chimeric antibody in which a mouse- or rat-derived antibody variable region is connected to a human-derived antibody constant region can be exemplified (Proc. Natl. Acad. Sci. USA, 81, 6851-6855, (1984)) .

As the humanized antibody, an antibody obtained by integrating only the complementarity-determining region (CDR) of a heterologous antibody into a human-derived antibody (Nature (1986) 321, pp. 522-525), and an antibody obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337) can be exemplified.

As the human antibody, an antibody generated by using a human antibody-producing mouse having a human chromosome fragment including genes of a heavy chain and light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology: Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.) can be exemplified. As an alternative, an antibody obtained by phage display, the antibody being selected from a human antibody library (see Wormstone, I. M. et. al, Investigative Ophthalmology & Visual Science. (2002) 43 (7), p.2301-2308; Carmen, S. et. al., Briefings in Functional Genomics and Proteomics (2002), 1 (2), p.189-203; Siriwardena, D. et. al., Ophthalmology (2002) 109 (3), p.427-431, etc.) can be exemplified.

In the present invention, modified variants of the anti-HER2 antibody used in the present invention are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by being expressed in a prokaryotic host cell. Further, an antibody labeled so as to enable the detection or isolation of the anti-HER2 antibody used in the present invention or the antigen, for example, an enzyme-labeled antibody, a fluorescence-labeled antibody, and an affinity-labeled antibody are also included in the meaning of the modified variant. Such a modified variant of the anti-HER2 antibody used in the present invention is useful for improving the stability and blood retention of the antibody, reducing the antigenicity thereof, detecting or isolating an antibody or an antigen, and so on.

Further, by regulating the modification of a glycan which is linked to the anti-HER2 antibody used in the present invention (glycosylation, defucosylation, etc.), it is possible to enhance antibody-dependent cellular cytotoxic activity. As the technique for regulating the modification of a glycan of antibodies, WO 99/54342, WO 00/61739, and WO 02/31140, etc. are known. However, the technique is not limited thereto. In the anti-HER2 antibody used in the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (the activation of complement, antibody-dependent cellular cytotoxicity, etc.) of the antibody. Therefore, in the anti-HER2 antibody used in the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variant having a deletion at the carboxyl terminus of the heavy chain of the anti-HER2 antibody used in the present invention is not limited to the above variants as long as the antigen-binding affinity and the effector function are conserved. The two heavy chains constituting the anti-HER2 antibody used in the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the anti-HER2 antibody used in the present invention and the culture conditions. However, an antibody in which one amino acid residue at the carboxyl terminus has been deleted in both of the two heavy chains in the anti-HER2 antibody used in the present invention can be preferably exemplified.

As isotypes of the anti-HER2 antibody used in the present invention, for example, IgG (IgG1, IgG2, IgG3, IgG4) can be exemplified, and IgG1 or IgG2 can be preferably exemplified.

Examples of the anti-HER2 antibody used in the present invention include trastuzumab (U.S. Patent No. 5821337) and pertuzumab (International Publication No. WO 01/00245), and trastuzumab can be preferably exemplified.

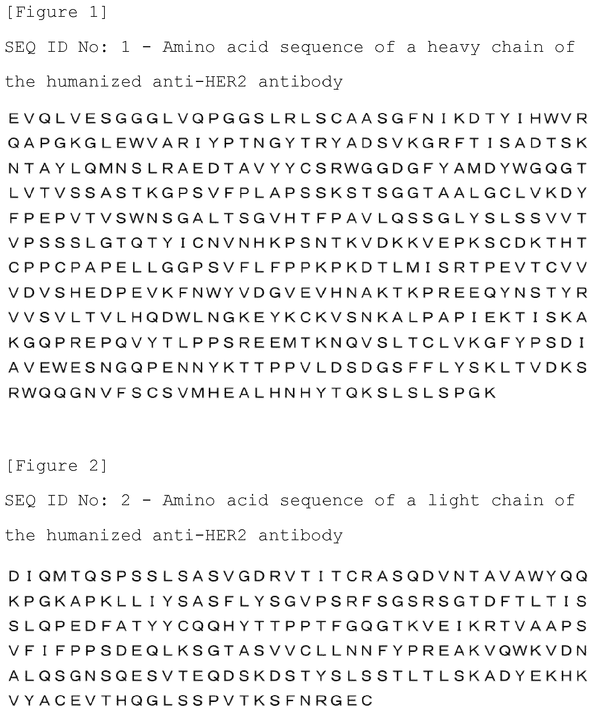

In the present invention, "trastuzumab" is a humanized anti-HER2 antibody comprising a heavy chain consisting of an amino acid sequence consisting of amino acid residues 1 to 449 of SEQ ID No: 1 (Figure 1) and a light chain consisting of an amino acid sequence consisting of amino acid residues 1 to 214 of SEQ ID No: 2 (Figure 2).

A preferred anti-HER2 antibody for use in the production of the anti-HER2 antibody-drug conjugate according to the present invention is:
(1) an antibody comprising a heavy chain consisting of an amino acid sequence consisting of amino acid residues 1 to 449 of SEQ ID No: 1 and a light chain consisting of an amino acid sequence consisting of amino acid residues 1 to 214 of SEQ ID No: 2; or
(2) an antibody comprising a heavy chain consisting of an amino acid sequence represented by SEQ ID No: 1 and a light chain consisting of an amino acid sequence represented by SEQ ID No: 2.

[Production of Anti-HER2 Antibody-Drug Conjugate]

A drug-linker intermediate for use in the production of the anti-HER2 antibody-drug conjugate according to the present invention is represented by the following formula.

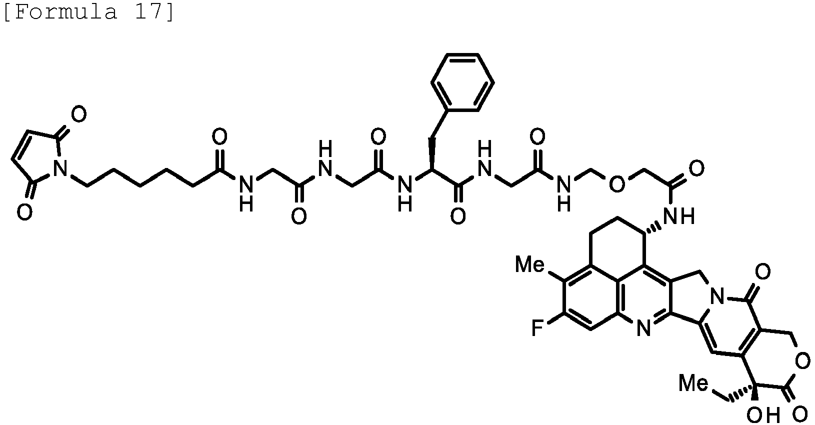

The drug-linker intermediate can be expressed as a chemical name, N-[6-(2,5-dioxo-2,5-dihydro-1H-pyrrole-1-yl)hexanoyl]glycylglycyl-L-phenylalanyl-N-[(2-{[(1S,9S)-9-ethyl-5-fluoro-9-hydroxy-4-methyl-10, 13-dioxo-2,3,9,10,13,15-hexahydro-1H,12H-benzo[de]pyrano[3',4' :6,7]indolizino[1,2-b]quinoline-1-yl]amino}-2-oxoethoxy)methyl]glycine amide and can be produced with reference to the description of International Publication No. WO 2015/115091.

The anti-HER2 antibody-drug conjugate used in the present invention can be produced by having the above-described drug-linker intermediate react with an anti-HER2 antibody having a thiol group (alternatively referred to as sulfhydryl group).

The anti-HER2 antibody having a sulfhydryl group can be obtained by a method well known to those skilled in the art (Hermanson, G.T, Bioconjugate Techniques, pp.56-136, pp.456-493, Academic Press (1996)). For example, by using 0.3 to 3 molar equivalent of a reducing agent such as tris(2-carboxyethyl)phosphine hydrochloride (TCEP) per interchain disulfide within the antibody and reacting with the anti-HER2 antibody in a buffer solution containing a chelating agent such as ethylenediaminetetraacetic acid (EDTA), an anti-HER2 antibody having a sulfhydryl group with partially or completely reduced interchain disulfides within the antibody can be obtained.

Further, by using 2 to 20 molar equivalents of the drug-linker intermediate per anti-HER2 antibody having a sulfhydryl group, an anti-HER2 antibody-drug conjugate in which 2 to 8 drug molecules are conjugated per antibody molecule can be produced.

The average number of conjugated drug molecules per antibody molecule of the anti-HER2 antibody-drug conjugate produced can be determined, for example, by a method of calculation based on measurement of UV absorbance for the anti-HER2 antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method).

Conjugation between the anti-HER2 antibody and the drug-linker intermediate, and calculation of the average number of conjugated drug molecules per antibody molecule of the anti-HER2 antibody-drug conjugate can be performed with reference to the descriptions in International Publication No. WO 2015/115091, and so on.

### [Therapeutic Agent and/or Method of Treatment]

The therapeutic agent and/or the method of treatment of the present invention comprise administering a specific anti-HER2 antibody-drug conjugate and can be used for treatment of HER2-mutated cancer.

The HER2-mutated cancer for which the therapeutic agent and/or the method of treatment of the present invention can be used is preferably at least one selected from the group consisting of lung cancer (including non-small cell lung cancer), breast cancer, gastric cancer (also referred to as gastric adenocarcinoma), colorectal cancer (also referred to as colon and rectal cancer and includes colon cancer and rectal cancer), esophageal cancer, salivary gland cancer, gastroesophageal junction adenocarcinoma, biliary tract cancer (including bile duct cancer), Paget's disease, pancreatic cancer, ovarian cancer, uterine carcinosarcoma, urothelial cancer, prostate cancer, bladder cancer, gastrointestinal stromal tumor, cervical cancer, squamous cell carcinoma, peritoneal cancer, liver cancer, hepatocellular carcinoma, endometrial cancer, kidney cancer, vulvar cancer, thyroid cancer, penis cancer, leukemia, malignant lymphoma, plasmacytoma, myeloma, head and neck cancer, pharyngeal cancer, glioblastoma multiforme, and melanoma, more preferably at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer, and uterine carcinosarcoma, even more preferably non-small cell lung cancer, and even more preferably unresectable and/or metastatic non-small cell lung cancer.

In the therapeutic agent and the method of treatment of the present invention, a dose per administration of the anti-HER2 antibody-drug conjugate used in the present invention is preferably in a range of 5.4 mg/kg (indicating that a dose per kg of the body weight is 5.4 mg, which applies to the following description) to 8 mg/kg, more preferably 5.4 mg/kg, 6.4 mg/kg, 7.4 mg/kg, or 8 mg/kg, even more preferably 5.4 mg/kg or 6.4 mg/kg, and even more preferably 6.4 mg/kg.

The therapeutic agent and the method of treatment of the present invention preferably comprises administering the anti-HER2 antibody-drug conjugate used in the present invention once every 3 weeks.

The therapeutic agent and the method of treatment of the present invention may contain one or more drugs (such as a second drug) other than the anti-HER2 antibody-drug conjugate used in the present invention. Thus, the therapeutic agent of the present invention or the anti-HER2 antibody-drug conjugate used in the present invention can be administered in combination with another drug, and the anti-cancer effect can be thereby enhanced. Another drug used for such a purpose may be administered to a subject simultaneously, separately, or sequentially with the anti-HER2 antibody-drug conjugate used in the present invention, or may be administered at different dosing intervals. Another drug or the second drug is preferably a cancer therapeutic agent. Such a cancer therapeutic agent is not limited as long as it is a drug having an antitumor activity, but examples thereof can include at least one selected from the group consisting of irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, tegafur-gimeracil-oteracil combination, cetuximab, panitumumab, bevacizumab, ramucirumab, regorafenib, trifluridine-tipiracil combination, gefitinib, erlotinib, afatinib, methotrexate, pemetrexed, trastuzumab emtansine, trastuzumab, pertuzumab, tamoxifen, toremifene, fulvestrant, leuprorelin, goserelin, letrozole, anastrozole, and progesterone formulation.

The therapeutic agent and the method of treatment of the present invention can be selectively used as an agent for drug therapy, which is a main method for treating cancer, and as a result, can delay development of cancer cells, inhibit growth thereof, and further kill cancer cells. These effects can allow cancer patients to be free from symptoms caused by cancer or achieve improvement in QOL of cancer patients and attain a therapeutic effect by sustaining the lives of cancer patients. Even if the therapeutic agent and the method of treatment of the present invention do not accomplish killing cancer cells, they can achieve higher QOL of cancer patients while achieving longer-term survival by inhibiting or controlling the growth of cancer cells.

In such a drug therapy, the therapeutic agent and the method of treatment of the present invention can be used alone, and in addition, they can be used in combination with an additional therapy in adjuvant therapy and can be combined with surgery, radiotherapy, hormone therapy, or the like. Furthermore, they can also be used for drug therapy in neoadjuvant therapy.

In addition to the therapeutic use as described above, for example, a prophylactic effect such as suppressing the growth of small metastatic cancer cells and further killing them can also be expected for the therapeutic agent and the method of treatment of the present invention. For example, an effect of inhibiting and killing cancer cells in a body fluid in the course of metastasis or an effect of, for example, inhibiting and killing small cancer cells immediately after implantation in any tissue can be expected. Accordingly, inhibition of cancer metastasis or a prophylactic effect can be expected, particularly, after surgical removal of cancer.

The therapeutic agent and the method of treatment of the present invention can be expected to exert a therapeutic effect by application as a systemic therapy to patients, and additionally, by local application to cancer tissues.

The therapeutic agent and the method of treatment of the present invention can preferably be used for mammals, and can more preferably be used for humans.

The therapeutic agent of the present invention can be administered as a pharmaceutical composition containing at least one pharmaceutically suitable ingredient. The pharmaceutically suitable ingredients can be appropriately selected and applied from formulation additives or the like that are generally used in the art, in view of the dosage, the administration concentration or the like of the anti-HER2 antibody-drug conjugate used in the present invention. For example, the therapeutic agent of the present invention can be administered as a pharmaceutical composition (hereinafter referred to as "pharmaceutical composition used in the present invention") containing a buffer such as histidine buffer, an excipient such as sucrose, and a surfactant such as polysorbate 80. The pharmaceutical composition used in the present invention can preferably be used as an injection, can more preferably be used as an aqueous injection or a lyophilized injection, and even more preferably be used as a lyophilized injection.

In the case that the pharmaceutical composition used in the present invention is an aqueous injection, it can preferably be diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

In the case that the pharmaceutical composition used in the present invention is a lyophilized injection, the composition is preferably dissolved in water for injection, subsequently a required amount can be diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

Examples of the administration routes which may be used to administer the pharmaceutical composition used in the present invention include intravenous, intradermal, subcutaneous, intramuscular, and intraperitoneal routes, and preferably include an intravenous route.

### Examples

The present invention is specifically described in view of the examples shown below. However, the present invention is not limited to these. Further, it is by no means to be interpreted in a limited way.

### Example 1: Production of Anti-HER2 Antibody-Drug Conjugate

In accordance with a production method described in International Publication No. WO 2015/115091 with use of a humanized anti-HER2 antibody (an antibody comprising a heavy chain consisting of the amino acid sequence consisting of amino acid residues 1 to 449 of SEQ ID No: 1 and a light chain consisting of the amino acid sequence consisting of amino acid residues 1 to 214 of SEQ ID No: 2), an anti-HER2 antibody-drug conjugate in which a drug-linker represented by the following formula: 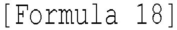 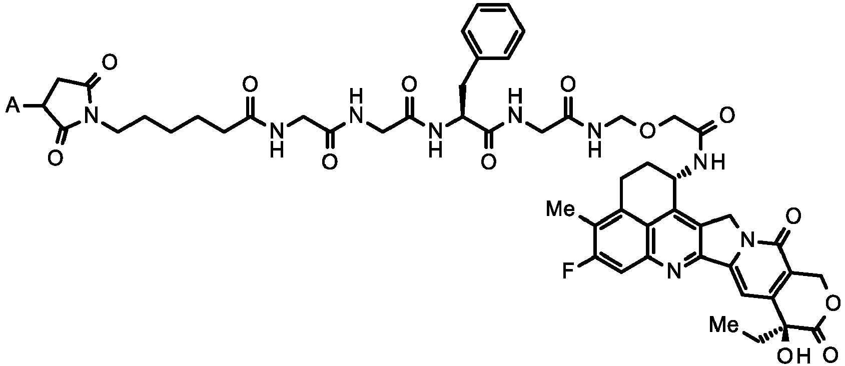 wherein A represents a connecting position to an antibody;
is conjugated to the anti-HER2 antibody via a thioether bond (referred to as "HER2-ADC (1)" in the present invention), was produced. The average number of units of the drugs conjugated per antibody molecule in HER2-ADC (1) is in the range of 7 to 8.

### Example 2: Clinical Study (Phase I Study)

The phase I study of HER2-ADC (1) was conducted on subjects with HER2-positive breast cancer (previously treated with trastuzumab emtansine), subjects with HER2-positive gastric cancer (previously treated with trastuzumab), subjects with HER2-low expressing breast cancer (with IHC 1+, or IHC 2+ and ISH -), and subjects with HER2-expressing (with IHC 1+ or more) or HER2-mutated (determined by IHC, FISH, NGS, or other methods) other solid cancer. The adverse events (AEs), the response rate (ORR: CR (complete response) + PR (partial response)), and the disease control rate (DCR: CR + PR + SD (stable disease)) were assessed.

From September 2015 to April 2018, HER2-ADC (1) was administered (6.4 mg/kg) to 12 subjects with HER2-expressing or HER2-mutated non-small cell lung cancer. The median value of the subjects' age was 58.5 years, and the median value of the subjects' previous treatment was 3 times. At the data cutoff, 8/12 (66.7%) of the subjects remained on treatment. The median value of the duration of treatment was 3.663 months. The ORR and DCR in the evaluable subjects were 62.5% (5/8) and 75.0% (6/8) respectively. Among them, 4 subjects were determined to have HER2 mutation, and the PR was 75.0% (3/4). The median value of the duration of response was 11.5 months. Tumor shrinkage was observed in 8/10 (80.0%) of the subjects subjected to one or more post-baseline scans (100% of the subjects showed tumor shrinkage at the first post-baseline scan (about 6 weeks after administration)). The reasons for treatment discontinuation were PD (3/4, 75.0%) and AE (1/4, 25.0%). Grade 3 or higher AEs were observed in 3/12 (25.0%) subjects. As major AEs, alopecia was observed at a proportion of 41.7% (grade 3 or higher was 0.0%), and fatigue was observed at a proportion of 41.7% (grade 3 or higher was 0.0%) (data as of April 18, 2018).

### Example 3: Clinical Study (Phase I Study)

Following Example 2, the phase I study of HER2-ADC (1) was conducted. So far, 18 subjects with HER2-expressing or HER2-mutated non-small cell lung cancer were subjected to administration of HER2-ADC (1) (6.4 mg/kg). The median value of the subjects' age was 58.0 years, and the median value of the subjects' previous treatment was 3 times. Out of the 18 subjects, 11 subjects were determined to have HER2 mutation (61.1%).

As the antitumor effects of HER2-ADC (1), Table 1 shows the objective response rate (ORR), the disease control rate (DCR), the duration of response (DoR), the time to response (TTR), and the progression-free survival (PFS) .

**[Table 1]**

| | Subjects with HER2-expressing or HER2-mutated non-small cell lung cancer (n = 18) | Subjects with HER2-mutated non-small cell lung cancer (n = 11) |
|---|---|---|
| Confirmed ORR | 58.8% (10/17) | 72.7% (8/11 ) |
| Confirmed DCR | 88.2% (15/17) | 100% (11/11) |
| DoR (Median value) | 9.9 months | 11.5 months |
| TTR (Median value) | 1.4 months | 1.4 months |
| PFS (Median value) | 14.1 months | 14.1 months |

In the cohort of subjects with HER2-expressing or HER2-mutated non-small cell lung cancer (18 subjects), HER2-ADC (1) showed an ORR of 58.8% (10/17) and a DCR of 88.2% (15/17). The median value of the DoR was 9.9 months, the median value of the TTR was 1.4 months, and the median value of the PFS was 14.1 months.

In the cohort of subjects with HER2-mutated non-small cell lung cancer (11 subjects), HER2-ADC (1) showed an ORR of 72.7% (8/11) and a DCR of 100% (11/11). The median value of the DoR was 11.5 months, the median value of the TTR was 1.4 months, and the median value of the PFS was 14.1 months. Further, Figure 3 shows maximum tumor shrinkages, and Figure 4 shows the time course of the tumor shrinkages.

Table 2 shows major adverse events (AE) observed in the treatment with HER2-ADC (1). All AEs were generally of low grade.

**[Table 2]**

| | Subjects with HER2-expressing or HER2-mutated non-small cell lung cancer (n = 18) | |
|---|---|---|
| | All grades | Grade 3 or higher |
| Nausea | 9 (50.0%) | 1 (5.6%) |
| Decreased appetite | 9 (50.0%) | 1 (5.6%) |
| Vomiting | 7 (38.9%) | 0 |
| Anemia | 2 (11.1%) | 0 |
| Alopecia | 9 (50.0%) | 0 |
| Fatigue | 8 (44.4%) | 0 |
| Diarrhea | 3 (16.7%) | 0 |
| Constipation | 4 (22.2%) | 0 |
| Platelet count decreased | 2 (11.1%) | 1 (5.6%) |
| Neutrophil count decreased | 2 (11.1%) | 2 (11.1%) |
| White blood cell count decreased | 2 (11.1%) | 1 (5.6%) |
| Malaise | 2 (11.1%) | 1 (5.6%) |
| Aspartate aminotransferase increased | 1 (5.6%) | 0 |

Other than the above, adverse events of special interest were interstitial lung disease observed in one patient (5.6%) and pneumonitis observed in one patient (5.6%) (data as of August 10, 2018).

### Example 4: Clinical Study (Phase II Study)

The phase II study of HER2-ADC (1) is conducted on subjects with HER2-overexpressing (with IHC 3+ or 2+) unresectable and/or metastatic non-small cell lung cancer (about 40 subjects: Cohort 1) and subjects with HER2-mutated unresectable and/or metastatic non-small cell lung cancer (about 40 subjects: Cohort 2). HER2-ADC (1) is administered at a dose of 6.4 mg/kg once every 3 weeks, to evaluate the response rate (ORR), the duration of response (DoR), the progression-free survival (PFS), and the overall survival (OS).

### Free Text of Sequence Listing

SEQ ID No: 1 - Amino acid sequence of a heavy chain of the humanized anti-HER2 antibody
SEQ ID No: 2 - Amino acid sequence of a light chain of the humanized anti-HER2 antibody
SEQ ID No: 3 - Amino acid sequence of HER2 protein
SEQ ID No: 4 - Nucleotide sequence of HER2 gene (cDNA)

## Claims

1. A therapeutic agent for HER2-mutated cancer comprising, as an active component, an anti-HER2 antibody-drug conjugate in which a drug-linker represented by the following formula: 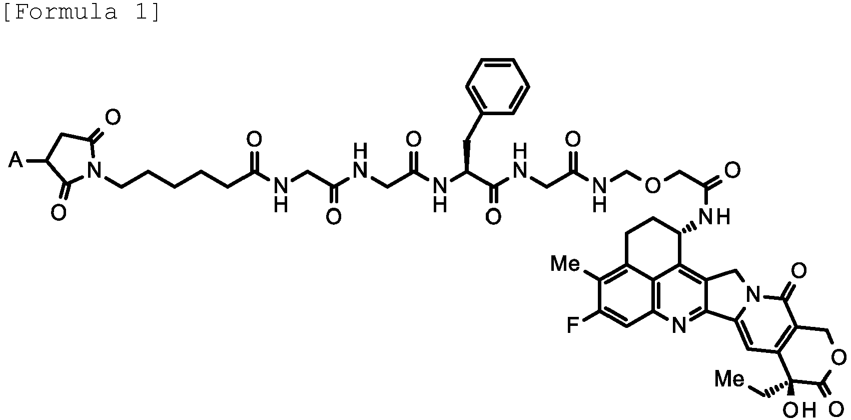 wherein A represents a connecting position to an anti-HER2 antibody;
is conjugated to the anti-HER2 antibody via a thioether bond.

2. The therapeutic agent according to claim 1, wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, 1613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770 _A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.

3. The therapeutic agent according to claim 2, wherein the HER2 mutation in the HER2-mutated cancer is at least one selected from the group consisting of Y772_A775dup, G778_P780dup, G776delinsVC, L755S, V777L, and S310F.

4. The therapeutic agent according to claim 1, wherein the HER2 mutation in the HER2-mutated cancer is an exon 20 insertion mutation.

5. The therapeutic agent according to claim 4, wherein the exon 20 insertion mutation is at least one selected from the group consisting of Y772_A775dup, G778_P780dup, G776delinsVC, E770_A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.

6. The therapeutic agent according to claim 5, wherein the exon 20 insertion mutation is at least one selected from the group consisting of Y772_A775dup, G778_P780dup, and G776delinsVC.

7. The therapeutic agent according to claim 1, wherein the HER2 mutation in the HER2-mutated cancer is a single base pair substitution mutation at a transmembrane domain of a HER2 protein.

8. The therapeutic agent according to claim 7, wherein the single base pair substitution mutation at the transmembrane domain of the HER2 protein is at least one selected from the group consisting of V659E, G660D, I654V, 1655V, and I661V.

9. The therapeutic agent according to claim 8, wherein the single base pair substitution mutation at the transmembrane domain of the HER2 protein is G660D.

10. The therapeutic agent according to claim 1, wherein the HER2 mutation in the HER2-mutated cancer is a single base pair substitution mutation at an extracellular domain of a HER2 protein.

11. The therapeutic agent according to claim 10, wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.

12. The therapeutic agent according to claim 11, wherein the single base pair substitution mutation at the extracellular domain of the HER2 protein is S310F.

13. The therapeutic agent according to any one of claims 1 to 12, wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.

14. The therapeutic agent according to any one of claims 1 to 12, wherein the cancer is non-small cell lung cancer.

15. The therapeutic agent according to claim 14, wherein the non-small cell lung cancer is unresectable and/or metastatic non-small cell lung cancer.

16. The therapeutic agent according to any one of claims 1 to 15, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

17. The therapeutic agent according to any one of claims 1 to 15, wherein the anti-HER2 antibody is an antibody comprising a heavy chain consisting of an amino acid sequence represented by SEQ ID NO: 1 and a light chain consisting of an amino acid sequence represented by SEQ ID NO: 2.

18. The therapeutic agent according to any one of claims 1 to 17, wherein the average number of units of the drug-linker conjugated per antibody in the anti-HER2 antibody-drug conjugate is in the range of from 7 to 8.

19. The therapeutic agent according to any one of claims 1 to 17, wherein the average number of units of the drug-linker conjugated per antibody in the anti-HER2 antibody-drug conjugate is in the range of from 7.5 to 8.

20. The therapeutic agent according to any one of claims 1 to 19, wherein a dose per administration of the anti-HER2 antibody-drug conjugate is in a range of 5.4 mg/kg to 8 mg/kg.

21. The therapeutic agent according to any one of claims 1 to 19, wherein a dose per administration of the anti-HER2 antibody-drug conjugate is 6.4 mg/kg.

22. The therapeutic agent according to any one of claims 1 to 21, wherein the anti-HER2 antibody-drug conjugate is administered once every 3 weeks.

23. A therapeutic agent for HER2-mutated cancer comprising, as an active component, an anti-HER2 antibody-drug conjugate represented by the following formula: 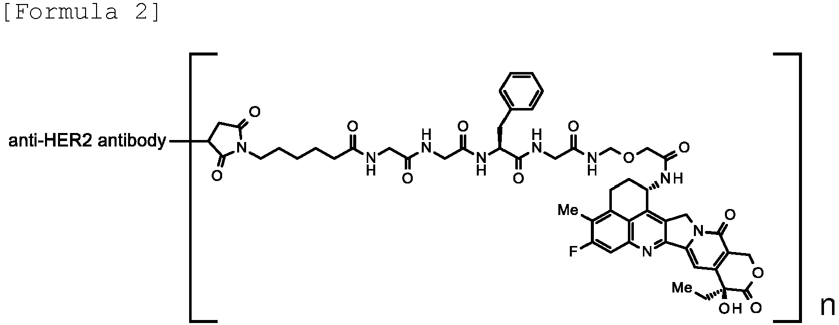 wherein the drug-linker is conjugated to the anti-HER2 antibody via a thioether bond, and n is the average number of units of the drug-linker conjugated per antibody.

24. The therapeutic agent according to claim 23, wherein the HER2 mutation in the HER2-mutated cancer is at least one selected from the group consisting of Y772_A775dup, G778_P780dup, G776delinsVC, L755S, V777L, V659E, G660D, S310F, A20T, A21S, R143Q, K200N, A242V, D277Y, A293P, N302K, V308M, S310Y, N319Y, S335C, R340P, S418T, W452C, V541M, 1613V, P627H, A644V, R647G, I654V, I655V, I661V, R678Q, Q680H, V697L, G704R, Q709L, Q711H, G727A, T733I, E744G, N745D, L755P, L755A, L755F, S760F, D769H, D769N, D769Y, E770 _A771insAYVM, A771_Y772insYVMA, M774_A775insAYVM, A775_G776insYVMA, A775G, G776delinsLC, G776C, G776delinsAVGC, G776delinsVV, G776_V777insL, G776L, G776_V777insVC, G776_V777insVGC, V777_G778insCG, V777_G778insG, G778_S779insG, S779P, S779_P780insVGS, P780_Y781insGSP, R784C, R784H, L785R, L786V, T791I, G804S, L807F, S819F, I829T, V842I, L846F, T862I, R868W, L869R, T875I, W906*, T917S, Q943*, S1007*, and S1151L.

25. The therapeutic agent according to claim 24, wherein the HER2 mutation in the HER2-mutated cancer is at least one selected from the group consisting of Y772_A775dup, G778_P780dup, G776delinsVC, L755S, V777L, and S310F.

26. The therapeutic agent according to claim 23, wherein the HER2 mutation in the HER2-mutated cancer is an exon 20 insertion mutation.

27. The therapeutic agent according to claim 26, wherein the exon 20 insertion mutation is at least one selected from the group consisting of Y772_A775dup, G778_P780dup, G776delinsVC, E770_A771insAYVM, A771_Y772insYVMA, M774_A775insAYVM, A775_G776insYVMA, G776delinsLC, G776delinsAVGC, G776delinsVV, G776_V777insL, G776_V777insVC, G776_V777insVGC, V777_G778insCG, V777_G778insG, G778_S779insG, S779_P780insVGS, and P780_Y781insGSP.

28. The therapeutic agent according to claim 27, wherein the exon 20 insertion mutation is at least one selected from the group consisting of Y772_A775dup, G778_P780dup, and G776delinsVC.

29. The therapeutic agent according to claim 23, wherein the HER2 mutation in the HER2-mutated cancer is a single base pair substitution mutation at a transmembrane domain of a HER2 protein.

30. The therapeutic agent according to claim 29, wherein the single base pair substitution mutation at the transmembrane domain of the HER2 protein is at least one selected from the group consisting of V659E, G660D, I654V, 1655V, and I661V.

31. The therapeutic agent according to claim 30, wherein the single base pair substitution mutation at the transmembrane domain of the HER2 protein is G660D.

32. The therapeutic agent according to claim 23, wherein the HER2 mutation in the HER2-mutated cancer is a single base pair substitution mutation at an extracellular domain of a HER2 protein.

33. The therapeutic agent according to claim 32, wherein the single base pair substitution mutation at the extracellular domain of the HER2 protein is at least one selected from the group consisting of S310F, A20T, A21S, R143Q, K200N, A242V, D277Y, A293P, N302K, V308M, S310Y, N319Y, S335C, R340P, S418T, W452C, V541M, I613V, P627H, A644V, and R647G.

34. The therapeutic agent according to claim 33, wherein the single base pair substitution mutation at the extracellular domain of the HER2 protein is S310F.

35. The therapeutic agent according to any one of claims 23 to 34, wherein the cancer in the HER2-mutated cancer is at least one selected from the group consisting of non-small cell lung cancer, breast cancer, gastric cancer, colorectal cancer, esophageal cancer, salivary gland cancer, gastroesophageal junction adenocarcinoma, biliary tract cancer, Paget's disease, pancreatic cancer, ovarian cancer and uterine carcinosarcoma.

36. The therapeutic agent according to any one of claims 23 to 34, wherein the cancer is non-small cell lung cancer.

37. The therapeutic agent according to claim 36, wherein the non-small cell lung cancer is unresectable and/or metastatic non-small cell lung cancer.

38. The therapeutic agent according to any one of claims 23 to 37, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

39. The therapeutic agent according to any one of claims 23 to 37, wherein the anti-HER2 antibody is an antibody comprising a heavy chain consisting of an amino acid sequence represented by SEQ ID NO: 1 and a light chain consisting of an amino acid sequence represented by SEQ ID NO: 2.

40. The therapeutic agent according to any one of claims 23 to 39, wherein the average number of units of the drug-linker conjugated per antibody in the anti-HER2 antibody-drug conjugate is in the range of from 7 to 8.

41. The therapeutic agent according to any one of claims 23 to 39, wherein the average number of units of the drug-linker conjugated per antibody in the anti-HER2 antibody-drug conjugate is in the range of from 7.5 to 8.

42. The therapeutic agent according to any one of claims 23 to 41, wherein a dose per administration of the anti-HER2 antibody-drug conjugate is in a range of 5.4 mg/kg to 8 mg/kg.

43. The therapeutic agent according to any one of claims 23 to 41, wherein a dose per administration of the anti-HER2 antibody-drug conjugate is 6.4 mg/kg.

44. The therapeutic agent according to any one of claims 23 to 43, wherein the anti-HER2 antibody-drug conjugate is administered once every 3 weeks.


SEQUENCE LISTING

<110>  DAIICHI SANKYO COMPANY, LIMITED

<120>  TREATMENT FOR HER2 MUTATED CANCER COMPRISING ADMINISTERING
       ANTI-HER2 ANTIBODY-DRUG CONJUGATE

<130>  DSPCT-FP1907

<150>  JP2018-101211
<151>  2018-05-28

<150>  JP2018-177132
<151>  2018-09-21

<160>  4

<170>  PatentIn version 3.5

<210>  1
<211>  450
<212>  PRT
<213>  Artificial Sequence

<220>
<223>  Heavy chain of humanized anti-HER2 antibody

<400>  1

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val

385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  2
<211>  214
<212>  PRT
<213>  Artificial Sequence

<220>
<223>  Light chain of humanized anti-HER2 antibody

<400>  2

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  3
<211>  1255
<212>  PRT
<213>  Homo sapiens

<400>  3

<210> 4
<211> 3768
<212> DNA
<213> Homo sapiens

<400> 4
atggagctgg cggccttgtg ccgctggggg ctcctcctcg ccctcttgcc ccccggagcc 60

gcgagcaccc aagtgtgcac cggcacagac atgaagctgc ggctccctgc cagtcccgag 120

acccacctgg acatgctccg ccacctctac cagggctgcc aggtggtgca gggaaacctg 180

gaactcacct acctgcccac caatgccagc ctgtccttcc tgcaggatat ccaggaggtg 240

cagggctacg tgctcatcgc tcacaaccaa gtgaggcagg tcccactgca gaggctgcgg 300

attgtgcgag gcacccagct ctttgaggac aactatgccc tggccgtgct agacaatgga 360

gacccgctga acaataccac ccctgtcaca ggggcctccc caggaggcct gcgggagctg 420

cagcttcgaa gcctcacaga gatcttgaaa ggaggggtct tgatccagcg gaacccccag 480

ctctgctacc aggacacgat tttgtggaag gacatcttcc acaagaacaa ccagctggct 540

ctcacactga tagacaccaa ccgctctcgg gcctgccacc cctgttctcc gatgtgtaag 600

ggctcccgct gctggggaga gagttctgag gattgtcaga gcctgacgcg cactgtctgt 660

gccggtggct gtgcccgctg caaggggcca ctgcccactg actgctgcca tgagcagtgt 720

gctgccggct gcacgggccc caagcactct gactgcctgg cctgcctcca cttcaaccac 780

agtggcatct gtgagctgca ctgcccagcc ctggtcacct acaacacaga cacgtttgag 840

tccatgccca atcccgaggg ccggtataca ttcggcgcca gctgtgtgac tgcctgtccc 900

tacaactacc tttctacgga cgtgggatcc tgcaccctcg tctgccccct gcacaaccaa 960

gaggtgacag cagaggatgg aacacagcgg tgtgagaagt gcagcaagcc ctgtgcccga 1020

gtgtgctatg gtctgggcat ggagcacttg cgagaggtga gggcagttac cagtgccaat 1080

atccaggagt ttgctggctg caagaagatc tttgggagcc tggcatttct gccggagagc 1140

tttgatgggg acccagcctc caacactgcc ccgctccagc cagagcagct ccaagtgttt 1200

gagactctgg aagagatcac aggttaccta tacatctcag catggccgga cagcctgcct 1260

gacctcagcg tcttccagaa cctgcaagta atccggggac gaattctgca caatggcgcc 1320

tactcgctga ccctgcaagg gctgggcatc agctggctgg ggctgcgctc actgagggaa 1380

ctgggcagtg gactggccct catccaccat aacacccacc tctgcttcgt gcacacggtg 1440

ccctgggacc agctctttcg gaacccgcac caagctctgc tccacactgc caaccggcca 1500

gaggacgagt gtgtgggcga gggcctggcc tgccaccagc tgtgcgcccg agggcactgc 1560

tggggtccag ggcccaccca gtgtgtcaac tgcagccagt tccttcgggg ccaggagtgc 1620

gtggaggaat gccgagtact gcaggggctc cccagggagt atgtgaatgc caggcactgt 1680

ttgccgtgcc accctgagtg tcagccccag aatggctcag tgacctgttt tggaccggag 1740

gctgaccagt gtgtggcctg tgcccactat aaggaccctc ccttctgcgt ggcccgctgc 1800

cccagcggtg tgaaacctga cctctcctac atgcccatct ggaagtttcc agatgaggag 1860

ggcgcatgcc agccttgccc catcaactgc acccactcct gtgtggacct ggatgacaag 1920

ggctgccccg ccgagcagag agccagccct ctgacgtcca tcatctctgc ggtggttggc 1980

attctgctgg tcgtggtctt gggggtggtc tttgggatcc tcatcaagcg acggcagcag 2040

aagatccgga agtacacgat gcggagactg ctgcaggaaa cggagctggt ggagccgctg 2100

acacctagcg gagcgatgcc caaccaggcg cagatgcgga tcctgaaaga gacggagctg 2160

aggaaggtga aggtgcttgg atctggcgct tttggcacag tctacaaggg catctggatc 2220

cctgatgggg agaatgtgaa aattccagtg gccatcaaag tgttgaggga aaacacatcc 2280

cccaaagcca acaaagaaat cttagacgaa gcatacgtga tggctggtgt gggctcccca 2340

tatgtctccc gccttctggg catctgcctg acatccacgg tgcagctggt gacacagctt 2400

atgccctatg gctgcctctt agaccatgtc cgggaaaacc gcggacgcct gggctcccag 2460

gacctgctga actggtgtat gcagattgcc aaggggatga gctacctgga ggatgtgcgg 2520

ctcgtacaca gggacttggc cgctcggaac gtgctggtca agagtcccaa ccatgtcaaa 2580

attacagact tcgggctggc tcggctgctg gacattgacg agacagagta ccatgcagat 2640

gggggcaagg tgcccatcaa gtggatggcg ctggagtcca ttctccgccg gcggttcacc 2700

caccagagtg atgtgtggag ttatggtgtg actgtgtggg agctgatgac ttttggggcc 2760

aaaccttacg atgggatccc agcccgggag atccctgacc tgctggaaaa gggggagcgg 2820

ctgccccagc cccccatctg caccattgat gtctacatga tcatggtcaa atgttggatg 2880

attgactctg aatgtcggcc aagattccgg gagttggtgt ctgaattctc ccgcatggcc 2940

agggaccccc agcgctttgt ggtcatccag aatgaggact tgggcccagc cagtcccttg 3000

gacagcacct tctaccgctc actgctggag gacgatgaca tgggggacct ggtggatgct 3060

gaggagtatc tggtacccca gcagggcttc ttctgtccag accctgcccc gggcgctggg 3120

ggcatggtcc accacaggca ccgcagctca tctaccagga gtggcggtgg ggacctgaca 3180

ctagggctgg agccctctga agaggaggcc cccaggtctc cactggcacc ctccgaaggg 3240

gctggctccg atgtatttga tggtgacctg ggaatggggg cagccaaggg gctgcaaagc 3300

ctccccacac atgaccccag ccctctacag cggtacagtg aggaccccac agtacccctg 3360

ccctctgaga ctgatggcta cgttgccccc ctgacctgca gcccccagcc tgaatatgtg 3420

aaccagccag atgttcggcc ccagccccct tcgccccgag agggccctct gcctgctgcc 3480

cgacctgctg gtgccactct ggaaaggccc aagactctct ccccagggaa gaatggggtc 3540

gtcaaagacg tttttgcctt tgggggtgcc gtggagaacc ccgagtactt gacaccccag 3600

ggaggagctg cccctcagcc ccaccctcct cctgccttca gcccagcctt cgacaacctc 3660

tattactggg accaggaccc accagagcgg ggggctccac ccagcacctt caaagggaca 3720

cctacggcag agaacccaga gtacctgggt ctggacgtgc cagtgtga 3768